(19) Europäisches Patentamt
European Patent Office
Office européen des brevets

(11) **EP 1 565 241 B1**

(12) **EUROPÄISCHE PATENTSCHRIFT**

(45) Veröffentlichungstag und Bekanntmachung des Hinweises auf die Patenterteilung:
**05.04.2006 Patentblatt 2006/14**

(21) Anmeldenummer: **03795849.3**

(22) Anmeldetag: **27.11.2003**

(51) Int Cl.:
**B01D 3/14** *(2006.01)*      **C07C 51/09** *(2006.01)*

(86) Internationale Anmeldenummer:
**PCT/EP2003/013384**

(87) Internationale Veröffentlichungsnummer:
**WO 2004/048308 (10.06.2004 Gazette 2004/24)**

(54) **VERFAHREN UND VORRICHTUNG ZUR HYDROLYTISCHEN GEWINNUNG EINER CARBONSÄURE UND ALKOHOL AUS DEM ENTSPRECHENDEN CARBONSÄUREESTER**

METHOD AND DEVICE FOR HYDROLYTICALLY OBTAINING A CARBOXYLIC ACID AND ALCOHOL FROM THE CORRESPONDING CARBOXYLIC ESTER

PROCEDE ET DISPOSITIF DE PRODUCTION HYDROLYTIQUE D'UN ACIDE CARBOXYLIQUE ET D'UN ALCOOL A PARTIR DE L'ESTER D'ACIDE CARBOXYLIQUE CORRESPONDANT

(84) Benannte Vertragsstaaten:
**AT BE BG CH CY CZ DE DK EE ES FI FR GB GR HU IE IT LI LU MC NL PT RO SE SI SK TR**

(30) Priorität: **28.11.2002 DE 10255648**

(43) Veröffentlichungstag der Anmeldung:
**24.08.2005 Patentblatt 2005/34**

(73) Patentinhaber: **Wacker Chemie AG 81737 München (DE)**

(72) Erfinder:
• **MICHL, Harald 84556 Kastl (DE)**
• **RAMGRABER, Franz 84489 Burghausen (DE)**

(74) Vertreter: **Renner, Thomas et al Wacker-Chemie GmbH Patente, Marken und Lizenzen Hans-Seidel-Platz 4 D-81727 München (DE)**

(56) Entgegenhaltungen:
**WO-A-01/27062**

**EP 1 565 241 B1**

## Beschreibung

Die Vorliegende Erfindung betrifft ein Verfahren sowie eine Vorrichtung zur verbesserten hydrolytischen Spaltung von Carbonsäureestern in die entsprechende Carbonsäure und Alkohol, wobei zumindest ein Teil des zur Hydrolyse notwendigen Wassers durch ein rückgeführtes Gemisch enthaltend Carbonsäure und Wasser ersetzt wird, das aus der Hydrolyse bereits im Verfahren umgesetzten Carbonsäureesters resultiert.

Die Umkehrung der Veresterung ist die Hydrolyse der Carbonsäureester in Carbonsäure und Alkohol. Die säurekatalysierte Hydrolyse ist wie die Veresterung selbst eine Gleichgewichtsreaktion. Carbonsäureester werden in der chemischen Industrie beispielsweise als Lösungsmittel oder Weichmacher eingesetzt oder fallen in verschiedenen Reaktionen als Neben- oder Hauptprodukte an. Beispielsweise ist Methylacetat ein typisches Nebenprodukt in der Herstellung von Polyvinylalkohol. Gemische enthaltend Methylacetat, die bei der Herstellung von Polyvinylalkohol anfallen, enthalten neben einer kleinen Menge von leichtsiedenden Substanzen wie beispielsweise Acetaldehyd, ein azeotropes Gemisch von Methylacetat und Methanol. Gemische der beschriebenen Zusammensetzung sind wirtschaftlich nicht oder nur schwer ohne weitere Aufreinigung oder Spaltung (Hydrolyse) der Carbonsäureester in die Edukte zu verwerten.

Die Hydrolyse der Carbonsäureester kann diskontinuierlich oder kontinuierlich durchgeführt werden. Hierzu eignen sich ein Reaktor in Verbindung mit einer konventionellen Destillation oder eine Reaktiv-Destillationskolonne. Die Verwendung eines Reaktors in Verbindung mit einer Destillationskolonne für die Hydrolyse von Methylacetat ist beispielsweise in der Patentschrift US 4,352,940 beschrieben. Das dort beschriebene Verfahren zur Hydrolyse von Methylacetat ist unter anderem nachteilhaft, da die Ausbeuten klein und mehrere Destillationsstufen nötig sind. Zudem treten Korrosionen durch die Verwendung von Schwefel- oder Salzsäure als homogene Katalysatoren auf.

Die Patentschrift US 5,113,015 beschreibt ein Verfahren zur Hydrolyse von Methylacetat, bei dem Methylacetat und Wasser mit einer Katalysatorpackung in einer Destillationskolonne in Kontakt gebracht werden, wodurch Methylacetat in Essigsäure und Methanol hydrolysiert und das Reaktionsgemisch in der Trennkolonne gleichzeitig zumindest teilweise aufgetrennt wird.

Die Patentschrift US 5,770,770 offenbart ebenfalls ein Verfahren zur Hydrolyse eines Methylacetatgemisches in einer Reaktiv-Destillationskolonne, wobei die Hydrolyse eines Methylacetatstroms enthaltend mindestens 50 Gew.-% Methylacetat in einer Reaktionszone aufweisend eine Ionentauscherpackung stattfindet. Das Methylacetatgemisch und Wasser werden in entgegengesetzter Richtung über die Ionentauscherpackung gefördert. Nicht umgesetztes Methylacetat und Wasser werden in die Reaktionszone rezykliert. Die Hydrolyseprodukte werden aus der Bodenfraktion abgetrennt. Die Verunreinigungen werden wieder in die Reaktionszone zurückgeführt.

Die Lehre der Patente, US 5,113,015 und US 5,770,770, ist die Verwendung von stark sauren Ionentauschern als Katalysatoren für die Hydrolyse von Carbonsäureestern. In der Patentschrift US 5,770,770 wird weiterhin beschrieben, das Ionentauschermaterial vorzugsweise in Form von Raschig-Ringen einzusetzen, wohingegen in US 5,113,015 das Katalysatormaterial vorzugsweise in Form von Matten verwendet wird. Als Katalysatorpackungsmaterial können nachgiebige, offenmaschige Stoffe, wie beispielsweise Drahtgewebe, starre, zelluläre Monolithe aus Stahl, Polymeren oder keramischem Material, sowie wellenförmigen Metall-, Kunststoff- oder Keramikplatten verwendet werden.

Die zuletzt beschriebenen Verfahren, welche nur eine einzige Reaktiv-Destillationskolonne verwenden, weisen den Nachteil auf, dass Methylacetat nur teilweise hydrolysiert wird. Der Eduktstrom bedingt den Einsatz von wenigstens einer zusätzlichen Reinigungsstufe. Weiterhin ist es nachteilhaft, dass Metallionen aus dem zuströmenden Methylacetat den Katalysator in der Reaktiv-Destillationskolonne deaktivieren, was zu geringen Standzeiten derartiger kontinuierlicher Anlagen führt.

In der europäischen Offenlegungsschrift EP 1 220 825 A2 wird ein Verfahren beschrieben, in dem der den Carbonsäureester enthaltende Zulaufstrom zuerst in einen Vorreaktor geleitet wird, in welchem der Carbonsäureester in Gegenwart von Wasser mit einem ersten Katalysator in Kontakt gebracht wird, wodurch der Carbonsäureester teilweise in die Hydrolyseprodukte gespalten wird. Das Reaktionsgemisch aus dem Vorreaktor wird in eine Reaktiv-Destillationskolonne geleitet und mit einem zweiten Hydrolyse-Katalysator kontaktiert zwecks wenigstens teilweiser Umsetzung des verbleibenden Carbonsäureesters in die entsprechende Carbonsäure und Alkohol.

Das in EP 1 220 825 beschriebene Verfahren hat den Vorteil, dass eine höhere Umsetzungsrate erzielt werden kann als bei den zuvor genannten Verfahren. Weiterhin ist durch die Verwendung eines Vorreaktors die Standzeit der Reaktiv-Destillationskolonne verbessert, da Katalysatorgifte, wie beispielsweise Metallionen, vornehmlich im Vorreaktor verbleiben. Die Wahl der Korngröße des eingesetzten Katalysatormaterials im Vorreaktor ist weniger eingeschränkt als für die in der Reaktiv-Destillationskolonne vorteilhaft eingesetzten Katalysatorpackungen.

Selbst wenn von einer erhöhten Rückführung von methylacetatreichem Kopfstrom der Reaktiv-Destillationskolonne auf den Vorreaktor ausgegangen wird, ist die Kapazität unter Verwendung einer bestehenden Struktur aufgrund der fixierten Geometrie des Reaktionsteils der Reaktiv-Destillationskolonne beschränkt.

Das in EP 1 220 825 beschriebenen Verfahren hat weiterhin den Nachteil, dass, wenn der Zustrom von Carbonsäureester den entsprechenden Alkohol enthält, das chemische Gleichgewicht der sauer katalysierten Hydrolyse

**EP 1 565 241 B1**

zu Ungunsten der Produkte im Reaktionsteils der Reaktiv-Destillationskolonne verschoben wird.

Die vorliegende Erfindung hat zur Aufgabe, ein verbessertes Verfahren sowie eine Vorrichtung zur sauer katalysierten Hydrolyse von Carbonsäureestern mittels einer Reaktiv-Destillationskolonne bereitzustellen. Weiterhin ist es Ziel dieser Erfindung, hohe Umsetzungsraten von Carbonsäureestern in Alkohol und Carbonsäure zu erzielen unter gleichzeitigem Einsatz von Mischungen des Carbonsäureesters mit dem entsprechenden Alkohol in variablen Zusammensetzungen.

Die Aufgabe dieser Erfindung wird gelöst durch ein Verfahren zur sauer katalysierten Hydrolyse eines Carbonsäureesters in die entsprechende Carbonsäure und den entsprechenden Alkohol in Gegenwart von Wasser, wobei ein Einlassstrom enthaltend den Carbonsäureester mit Wasser vermischt in einen ersten Vorreaktor aufweisend einen Hydrolyse-Katalysator geleitet wird, wodurch der Carbonsäureester zumindest teilweise in die Hydrolyseprodukte gespalten wird, das Reaktionsgemisch aus dem ersten Vorreaktor abgezogen und wenigstens teilweise in eine Reaktiv-Destillationskolonne enthaltend einen Hydrolyse-Katalysator geleitet wird, wodurch der Carbonsäureesterstrom enthaltend Wasser weiter in Carbonsäure und Alkohol umgesetzt und zeitgleich wenigstens teilweise in die Komponenten getrennt wird, wobei die leichter flüchtigen Verbindungen bzw. leichtsiedenden Carbonsäureester enthaltenden Mischungen zumindest teilweise als Destillat, beispielsweise als Kopffraktion, abgezogen werden und die schwerer flüchtigen Verbindungen sich wenigstens teilweise als Bodenfraktion sammeln, die in eine weitere Destillationskolonne geleitet wird, dadurch gekennzeichnet, dass die wässrige Carbonsäure aus der Bodenfraktion oder aus der unteren Rektifikationszone der Destillationskolonne teilweise mit weiterem Carbonsäureester vermischt einem zweiten Vorreaktor enthaltend einen Hydrolyse-Katalysator zugeführt wird, das Reaktionsgemisch aus diesem zweiten Vorreaktor abgezogen und wenigstens teilweise in die Reaktiv-Destillationskolonne geführt wird.

Als Destillat der Reaktiv-Destillationskolonne wird hiermit jede der Reaktiv-Destillationskolonne oberhalb der Reaktionszone inklusive dem vollständigen Kondensationssystem der Reaktiv-Destillationskolonne entnommene Fraktion, wie beispielsweise ein Seitenabzug oder eine durch Partialkondensation entnommene Fraktion, ein Teilstrom oder die vollständige Kopffraktion sowie Fraktionen derselben, definiert.

Vorteilhaft wird zumindest ein Teil der Bodenfraktion der Reaktiv-Destillationskolonne wenigstens einer weiteren Destillationskolonne zugeführt und wenigstens teilweise in die Komponenten aufgetrennt. Eine oder mehrere nachgeschaltete Trennstufen werden vorzugsweise dann eingesetzt, wenn das resultierende Reaktionsgemisch möglichst vollständig in die einzelnen Komponenten getrennt werden soll. Die Bodenfraktion oder eine Fraktion aus der unteren Rektifikationszone, vorzugsweise eine Fraktion aus der unteren Hälfte der Rektifikationszone dieser Destillationskolonne enthaltend die resultierende Carbonsäure und Wasser werden zumindest teilweise dem zweiten Vorreaktor zugeführt, wobei diese zuvor mit neu zugeführten Carbonsäureester oder mit dem direkt anfallenden oder mittels weiterer Trennschritte an Carbonsäureester aufkonzentrierten Destillat der Reaktiv-Destillationskolonne enthaltend noch nicht umgesetzten Carbonsäureester, das ursprünglich dem Einlass des ersten Vorreaktors zugeführt wurde, gemischt werden können.

Vorteilhaft ist bei der Mischung der wässrigen Carbonsäure aus der Bodenfraktion der Destillationskolonne oder einer Fraktion aus der unteren Rektifikationszone, dass dieser Strom bereits eine erhöhte Temperatur von 50 bis 150 °C aufweist und beim Vermischen mit dem Strom enthaltend Carbonsäureester vor Eintritt in den zweiten Vorreaktor die resultierende Mischung bereits auf Temperaturen von 50 bis 100°C erwärmt. Somit ist es nicht notwendig, dem Verfahren zur Erwärmung des Zulaufs des zweiten Vorreaktors erneut Energie in Form von Wärme zuzuführen.

Vorteilhaft kann der Carbonsäureesterstrom auf den Gesamtprozeß, der einem oder beiden Vorreaktoren zugeführt wird, mit wenigstens einer aequimolaren Menge Wasser vermischt werden. Zweckmäßigerweise liegt das molare Verhältnis zwischen Carbonsäureester und Wasser zwischen 1:1 und 1:15, vorzugsweise zwischen 1:2 und 1:10 und besonders bevorzugt zwischen 1:4 und 1:8.

Die Hydrolysereaktion kann auch bei Raumtemperatur durchgeführt werden. Bevorzugt wird das Gemisch aus Carbonsäureester und Wasser auf eine Temperatur zwischen 30 und 120°C, besonders bevorzugt auf 50 bis 100°C erwärmt, da das Hydrolysegleichgewicht bei erhöhten Temperaturen vorteilhaft auf die Seite der Produkte Carbonsäure und Alkohol verschoben und die Reaktionsgeschwindigkeit gesteigert wird. Die Auswahl der Temperatur wird wesentlich von der thermischen Stabilität des Ionenaustauschers geprägt.

Besonders vorteilhaft wird die Reaktiv-Destillationskolonne so gefahren, dass die Carbonsäure und wenigstens ein Teil des Wassers und des Alkohols in der Bodenfraktion der Reaktiv-Destillationskolonne zurückbleiben. Dabei erfolgt die Einleitung des Reaktionsgemisches aus dem ersten Vorreaktor in die Reaktiv-Destillationskolonne vorzugsweise an einem Punkt im oberen Drittel der Katalysatorzone oder oberhalb der Katalysatorzone und unterhalb der oberen Rektifikationszone in die Reaktiv-Destillationskolonne. Im Falle der Hydrolyse höhersiedender Carbonsäuren, die ein leichtsiedendes wasserreiches, meist ternäres Azeotrop ausbilden, wie beispielsweise Isobutylacetat, empfiehlt sich vorteilhaft die Einspeisung an einem Punkt unterhalb der Reaktionszone und oberhalb der unteren Rektifikationszone oder im oberen Drittel der unteren Rektifikationszone. Das Reaktionsgemisch aus dem zweiten Vorreaktor wird vorteilhaft unterhalb der Reaktionszone und oberhalb der unteren Rektifikationszone oder im Falle der Hydrolyse höhersiedender Carbonsäuren, die ein wasserreiches, meist ternäres Azeotrop ausbilden vorteilhaft im oberen Drittel der unteren Rek-

3

tifikationzone eingespeist. Die anfallende Bodenfraktion, welche beispielsweise Alkohol, Wasser und Carbonsäure enthält, wird einer weiteren Destillationskolonne zugeführt, in welcher das Gemisch weiter aufgetrennt wird.

Das Destillat der Reaktiv-Destillationskolonne kann direkt oder nach Durchlaufen weiterer Trenn- oder Destillationsstufen teilweise dem ersten Vorreaktor, dem zweiten Vorreaktor oder beiden Vorreaktoren zugeführt werden, um den im Destillat gegebenenfalls noch enthaltenen Carbonsäureester weiter umzusetzen. Eine solche Rückführung des Destillats der Reaktiv-Destillationskolonne erlaubt die Kapazität der Anlage wesentlich zu steigern.

Aufgrund des üblicherweise ansteigenden Gehalts an leichtsiedenden Verunreinigungen, die am Kopf der Reaktiv-Destillationskolonne zumindest teilweise ausgeschleust werden, und des zur Katalysatorzone hin ansteigenden Wassergehalts bei einem vorzugsweise oberhalb der Katalysatorzone angeordneten Zulaufs in die Reaktiv-Destillationskolonne ist es vorteilhaft, das Destillat der oberen Rektifikationszone, die oberhalb der Katalysatorzone angeordnet ist, vorzugsweise in deren unterem Drittel, zu entnehmen.

Das erfindungsgemäße Verfahren wird vorteilhaft kontinuierlich betrieben, d.h. Carbonsäureester und Wasser werden kontinuierlich in den ersten Vorreaktor geleitet und das resultierende Reaktionsgemisch kontinuierlich aus dem ersten Vorreaktor abgezogen und in die Reaktiv-Destillationskolonne geleitet, in der nicht umgesetzter Carbonsäureester größtenteils in seine Hydrolyseprodukte umgesetzt wird, wobei die Hydrolyseprodukte kontinuierlich im Destillat, in der Kopffraktion und in der Bodenfraktion abgezogen werden und vorzugsweise das Destillat direkt oder nach dem Durchlaufen weiterer Trenn- oder Destillationsstufen zur Aufkonzentrierung des Carbonsäureesters kontinuierlich erneut dem ersten Vorreaktor, dem zweiten Vorreaktor oder beiden Vorreaktoren zugeführt, die Bodenfraktion ebenfalls kontinuierlich einer Destillationskolonne zugeführt und die wässrige Carbonsäure in der Bodenfraktion oder einer Fraktion aus der unteren Rektifikationszone, vorzugsweise aus der unteren Hälfte der unteren Rektifikationszone, dieser Destillationskolonne vermischt mit Carbonsäureester zumindest teilweise dem zweiten Vorreaktor zugeführt wird. Der zugeführte Volumenstrom pro Einheitsvolumen Hydrolyse-Katalysator in beiden Vorreaktoren liegt vorteilhaft zwischen 0,1 - 15 $h^{-1}$, vorzugsweise 0,5 - 8 $h^{-1}$ und besonders bevorzugt zwischen 1 - 4 $h^{-1}$.

Gegenstand der vorliegenden Erfindung ist auch eine Vorrichtung zur sauer katalysierten Hydrolyse eines Carbonsäureesters in die entsprechende Carbonsäure und den entsprechenden Alkohol in Gegenwart von Wasser, enthaltend

a) einen ersten Vorreaktor **1** enthaltend einen Hydrolyse-Katalysator mit wenigstens einer Einlassleitung **2** zur Einspeisung eines Fluidstroms enthaltend den Carbonsäureester, Leitung **2'**, und Wasser, Leitung **2"**, sowie wenigstens einem Auslass **4,** zur Abführung des Reaktionsgemisches,

b) wenigstens eine Heizvorrichtung zum Heizen des Einlassstroms, Leitung **2,** des ersten Vorreaktors **1,** oder von beiden,

c) einen zweiten Vorreaktor **19** enthaltend einen Hydrolyse-Katalysator mit mindestens einem Einlass für einen Fluidstrom enthaltend zumindest teilweise die wässrige Carbonsäure aus der Bodenfraktion oder einer Fraktion aus der unteren Rektifikationszone **14** der Destillationskolonne **13,** Leitung **18,** weiterhin vermischt mit einem Carbonsäureesterstrom, Leitung **20,** und mindestens einem Auslass, Leitung **25,**

d) eine Reaktiv-Destillationskolonne **7** enthaltend eine Katalysatorzone **8** aufweisend einen Hydrolyse-Katalysator mit einem Einlass **6** verbunden mit dem ersten Vorreaktor **1,** einem Einlass verbunden mit dem zweiten Vorreaktor, Leitung **25,** wobei die Katalysatorzone **8** zwischen einer unteren Rektifikationszone **9** und einer oberen Rektifikationszone **10** angeordnet ist,

e) am Destillationskopf oder am Kondensationssystem der Reaktiv-Destillationskolonne angeschlossene Leitung **22** zur Abführung der Kopffraktion oder als Purge,

f) an der die Kopffraktion der Reaktiv-Destillationskolonne **7** führende Leitung **22** oder am oberen Destillationsteil **10** inklusive dem vollständigen Kondensationssystem der Reaktiv-Destillationskolonne **7** anbindende Leitung **21** zur Abführung von Destillat

g) am Destillationsfuß der Reaktiv-Destillationskolonne **7** angeschlossene Leitung **11** zum Abziehen der Bodenfraktion,

h) eine Destillationskolonne **13,** wobei die Leitung **11** zum Abziehen der Bodenfraktion der Reaktiv-Destillationskolonne **7** den Einlass bildet, an deren Destillationsfuß, Leitung **26,** wässrige Carbonsäure abgezogen wird,

i) Leitung **18,** führend eine Fraktion entnommen aus der unteren Rektifikationszone **14** der Destillationskolonne **13** oder eine Teilmenge der Leitung **26** enthaltend wässrige Carbonsäure, wobei diese mit Carbonsäureester, Leitung **20,** vermischt den Zufluss auf den zweiten Vorreaktor **19** bildet.

In einer bevorzugten Ausführungsform weisen der erste oder zweite Vorrektor unabhängig von einander zwei Reaktionskammern auf. Weiterhin können Mittel vorgesehen sein, um den Zustrom jeweils durch eine der Reaktionskammern leiten zu können, so dass die jeweils andere Reaktionskammer mit frischem Katalysator beschickt werden kann. Dies hat den Vorteil, dass die Einrichtung über einen langen Zeitraum kontinuierlich betrieben werden kann. In einer weiteren bevorzugten Ausführungsform weisen der erste oder zweite Vorreaktor unabhängig von einander einen Bypass auf, um ebenfalls den Austausch der Katalysatorpackungen ohne Unterbrechung des kontinuierlichen Betriebes der Vorrichtung zu gewährleisten.

Bevorzugt weist die Reaktiv-Destillationskolonne **7** eine Katalysatorzone **8** sowie eine untere (**9**) und eine obere Rektifikationszone **10** auf, wobei die Katalysatorzone **8** zwischen der unteren und der oberen Rektifikationszone (**9,10**) angeordnet ist. Die Rektifikationszonen können Trennböden, Raschig-Ringe, strukturierte Stoffaustauschpackungen oder ähnliches aufweisen.

Der erste Vorreaktor **1,** der zweite Vorreaktor **19** oder die Reaktiv-Destillationskolonne **7** sind vorteilhaft unabhängig voneinander als Rohr ausgebildet. In diesem kann ein Hydrolyse-Katalysator aufgeschüttet oder in einer strukturierten Katalysatorpackung angeordnet sein. Zweckmäßigerweise sind die Hydrolyse-Katalysatoren saure Feststoffkatalysatoren, wobei eine Korngröße zwischen ungefähr 0,3 und 3 mm, vorzugsweise zwischen 0,6 und 1,2 mm bevorzugt wird. Während die Hydrolyse-Katalysatoren in den Vorreaktoren (**1, 19**) vorzugsweise in Form von Kugeln, Ringen, Strangpresslingen etc. als Schüttung vorliegen, ist der Hydrolyse-Katalysator der Reaktiv-Destillationskolonne **7** vorteilhaft als strukturierte Katalysatorpackung eingebracht. Geeignete strukturierte Katalysatorpackungen sind beispielsweise in den Patentschriften US 5,417,939, US 5,470,542 und US 5, 536, 699. beschrieben, deren diesbezügliche Offenbarung hiermit durch Bezugnahme aufgenommen wird. Als strukturierte Katalysatorpackung soll eine Struktur mit Rückhalteeinrichtungen (z.B. Taschen) für festes Katalysatormaterial und mit Strömungskanälen, welche in der Struktur vorgesehen sind, verstanden werden. Denkbar ist sowohl in den Vorreaktoren (**1, 19**) als auch in der Reaktiv-Destillationskolonne **7** solche strukturierte Katalysatorpackungen zu verwenden. Weiterhin können die Vorreaktoren (**1, 19**) beispielsweise in Form von Rührwerken mit eingebrachten oder aufgewirbelten Katalysator ausgebildet werden. Die Reaktiv-Destillationskolonne **7** kann als Bodenkolonnen gewählt werden, wobei der Ionenaustauscher als Katalysator auf den Böden selbst und in den Ablaufschächten angeordnet werden kann.

In einer weiteren bevorzugten Ausführungsform wird die Einlassleitung **2** auf den ersten Vorreaktor dadurch beheizt, dass der Carbonsäureesterstrom, Leitung **2',** mit heißem Wasser, Leitung **2",** beispielsweise Heizdampfkondensat vermischt wird, um so die gewünschte Reaktionstemperatur einzustellen. In dieser bevorzugten Ausführungsform kann dann auf eine weitere Heizvorrichtung des Zulaufes des ersten Vorreaktors sowie des Vorreaktors selbst verzichtet werden.

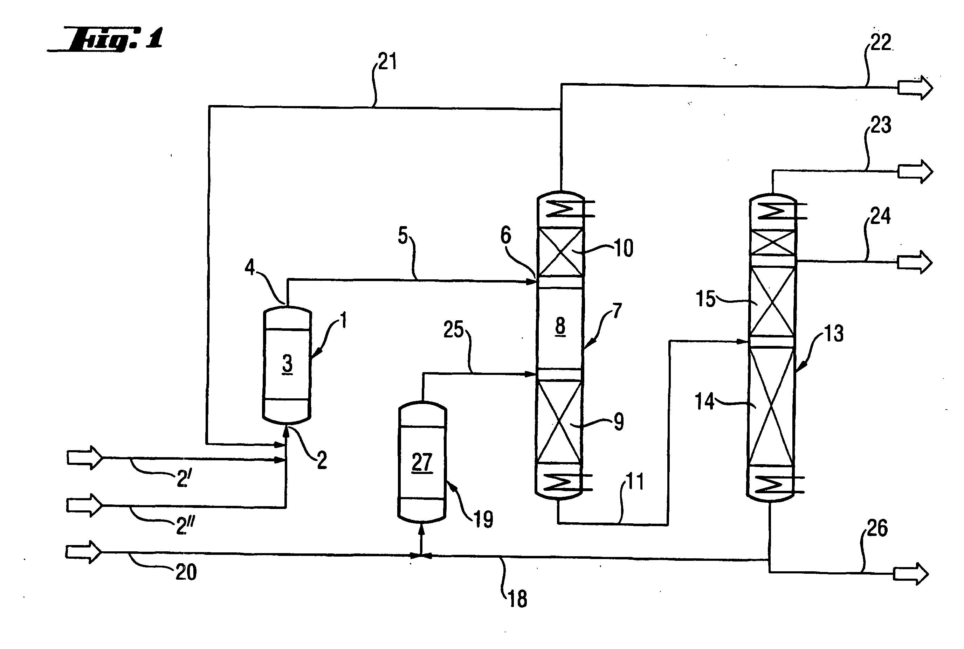

Figur 1 zeigt eine schematische Darstellung einer bevorzugten Ausführung der erfindungsgemäßen Vorrichtung. Der erste Vorreaktor **1** besitzt eine Einlassleitung **2** zum Einleiten eines Fluidstroms in den Reaktorraum **3** enthaltend den Hydrolyse-Katalysator, vorzugsweise einen sauren Feststoffkatalysator, und einen Auslass **4,** zum Abziehen des Reaktionsgemisches. Die Verbindungsleitung **5** verbindet den Auslass **4** des ersten Vorreaktors mit einem Einlass **6** an der Reaktiv-Destillationskolonne **7.** Der erste Vorreaktor **1** ist vorzugsweise rohrförmig, wobei die Einlassleitung **2** gespeist wird aus der Zuleitung des zu hydrolysierenden Carbonsäureestergemisches, Leitung **2',** und der Zuleitung des Wassers, Leitung **2".** Der erste Vorreaktor **1** weist einen Hydrolyse-Katalysator auf, der im Reaktorraum **3** angeordnet ist.

Die Reaktiv-Destillationskolonne **7** besitzt eine Katalysatorzone **8** und eine untere Rektifikationszone **9** sowie eine obere Rektifikationszone **10,** welche unterhalb bzw. oberhalb der Katalysatorzone **8** angeordnet sind. In der Katalysatorzone **8** ist ein Hydrolyse-Katalysator, vorzugsweise ein saurer Feststoffkatalysator besonders bevorzugt in einer strukturierten Katalysatorpackung, enthalten. Die Rektifikationszonen sind beispielsweise durch Raschig-Ringe, Kolonnenböden, strukturierte (Stoffaustausch-)Packungen oder ähnliches in bekannter Weise gebildet. Am Kolonnenfuß der unteren Rektifikationszone **9** ist eine Leitung **11** zum Abziehen der Bodenfraktion und am Kolonnenkopf der oberen Rektifikationszone **10** eine Leitung **22** zum Abziehen der Kopffraktion der Reaktiv-Destillationskolonne **7** vorgesehen. Die Zuführung des Produktes des ersten Vorreaktors **1,** Leitung **5,** erfolgt vorzugsweise oberhalb der Reaktionszone **8** und unterhalb der oberen Rektifikationszone **10** oder im oberen Drittel der Reaktionszone **8.** Im Falle höher siedender Carbonsäureester, die ein leichtsiedendes wasserreiches, meist ternäres Azeotrop bilden, ist eine Einspeisung unterhalb der Reaktionszone **8** und oberhalb der unteren Rektifikationszone **9** oder im oberen Drittel der unteren Rektifikationszone **9** vorteilhaft.

Die Leitung **11** ist mit einer Destillationskolonne **13** verbunden. Die Destillationskolonne **13** besitzt vorzugsweise eine untere Rektifikationszone **14** und eine obere Rektifikationszone **15.** Die Leitung **11** wird vorzugsweise zwischen den Rektifikationszonen **14** und **15** der Destillationskolonne **13** angeschlossen. Der Destillationskolonne **13** wird das Reaktionsprodukt wässrige Carbonsäure als Bodenfraktion entnommen und über die Leitung **26** abgeleitet. Ein Teil der wässrigen Carbonsäure wird entweder der Leitung **26,** dem Kolonnenfuß der Destillationskolonne **13,** oder der unteren Rektifikationszone **14,** vorzugsweise der unteren Hälfte der unteren Rektifikationszone **14,** entnommen und zumindest teilweise über die Leitung **18** dem zweiten Vorreaktor **19** zugeführt. Vor Eintritt in den zweiten Vorreaktor **19** mit dem mit Hydrolyse-Katalysator, vorzugsweise ein saurer Feststoffkatalysator, gefüllten Reaktionsraum **27** wird der Leitung **18** über die Leitung **20** weiterer Carbonsäureester zugeführt.

Der zweite Vorreaktor weist eine Katalysatorzone **27** auf, die vorzugsweise einen sauren Feststoffkatalysator enthält. Der zweite Vorreaktor wird vorzugsweise von unten nach oben durchströmt. Der Auslass des zweiten Vorreaktors ist mittels der Leitung **25** mit der Reaktiv-Destillationskolonne **7** verbunden. Die Leitung **25** wird hierbei vorzugsweise unterhalb der Katalysatorzone **8** und oberhalb der unteren Rektifikationszone **9** in die Reaktiv-Destillationskolonne **7** geführt. Für den Fall der Hydrolyse höher siedender Carbonsäureester mit einem hohen Wasseranteil im ternären Azeotrop kann der Zulauf vom zweiten Vorreaktor in die Hydrolysekolonne durch das Einleiten in das obere Drittel der unteren Rektifikationszone **9** gegebenenfalls optimiert werden.

Die Leitung **22** zum Abziehen der Kopffraktion der Reaktiv-Destillationskolonne **7** enthaltend nicht umgesetzten Carbonsäureester kann über die Leitung **21** mit der Leitung **2,** Leitung **2'** oder Leitung **2"** des zu hydrolysierenden Carbonsäureestergemisches verbunden werden. Ein Teil der Kopffraktion der Reaktiv-Destillationskolonne **7** wird dabei über die Leitung **22** entnommen, um der Vorrichtung Leichtsieder und Inerte zu entziehen (Purge). Damit wird auch eine sicherheitstechnisch bedenkliche Ansammlung von Leichtsiedern, wie beispielsweise Acetaldehyd, vermieden.

Der in der Reaktiv-Destillationskolonne **7** nicht umgesetzte Carbonsäureester wird in einer bevorzugten Ausführungsform mittels Leitung **21** nicht der Leitung **22,** sondern der oberen Rektifikationszone der Reaktiv-Destillationskolonne **7** entnommen.

Der entstehende bzw. freigesetzte Alkohol wird vorzugsweise an der oberen Rektifikationszone **15** der Destillationskolonne **13** mittels der Leitungen **23** bzw. **24** entnommen. Der Vorteil der Entnahme des Alkohols über Leitung **24** ist, dass man das mit Carbonsäureester angereicherte Destillat der Leitung **23** der Destillationskolonne **13** in die Reaktiv-Destillationskolonne **7** recyclieren kann und dort bevorzugt unterhalb der Katalysatorzone **8** und oberhalb der unteren Rektifikationszone **9** oder im oberen Drittel der unteren Rektifikationszone **9** einspeist. Eine Rückführung in den Prozess durch Einbinden der Leitung **23** in den ersten Vorreaktor **1** und in den zweiten Vorreaktor **19** ist ebenfalls möglich.

Um ein Teilverdampfen des Zuflusses auf den zweiten Vorreaktor **19** zu vermeiden, ist es vorteilhaft, das Mischen der wässrigen Carbonsäure und des carbonsäureesterhaltigen Zuflusses sowie die Umsetzung im zweiten Vorreaktor **19** unter Überdruck erfolgen zu lassen. Der erforderliche Überdruck richtet sich nach dem Mischungsverhältnis und dem Stoffsystem, bevorzugt sind Drücke im Bereich 1 bis 6 bar.

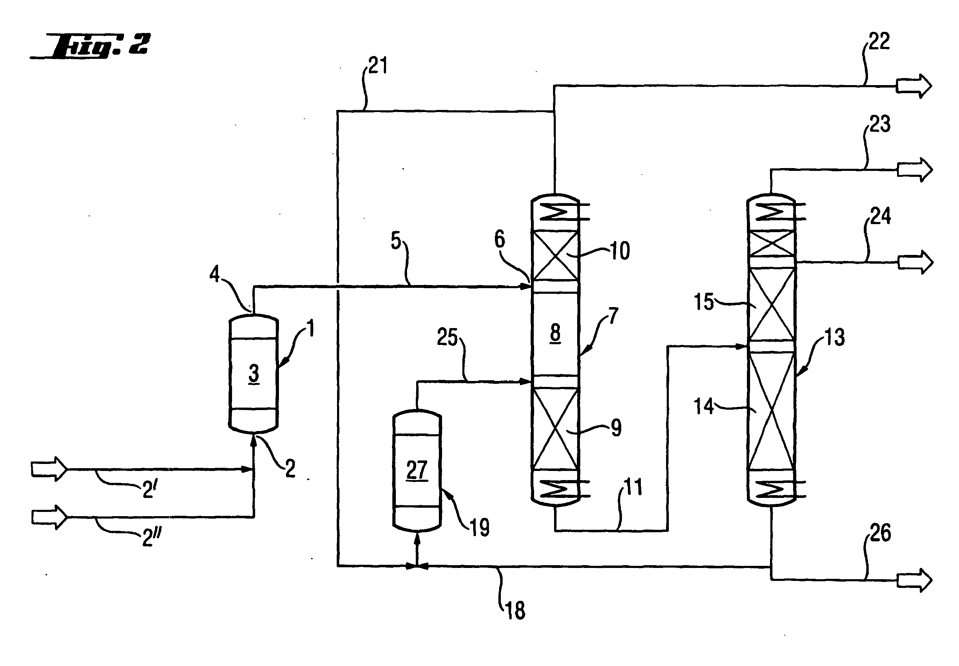

Eine zweite bevorzugte Ausführungsform der erfindungsgemäßen Vorrichtung ist in Figur 2 wiedergegeben. Die erfindungsgemäße Vorrichtung gemäß Zeichnung 2 unterscheidet sich von der erfindungsgemäßen Vorrichtung in Figur 1 darin, dass die Leitung **21** nicht mit der Zuleitung des zu hydrolysierenden Carbonsäureestergemisches **2',** der Wasser-Zuleitung **2"** oder dem Gemisch **2,** sondern mit der Leitung **18,** die die wässrige Carbonsäure aus der Bodenfraktion der Destillationskolonne **13** in den zweiten Vorreaktor **19** führt, verbunden ist. Die Zuleitung **20** entfällt entsprechend, wobei die Leitung **21** mit der Leitung **22** verbunden sein kann oder in einer weiteren bevorzugten Ausführungsform der oberen Rektifikationszone **10** entnommen werden kann.

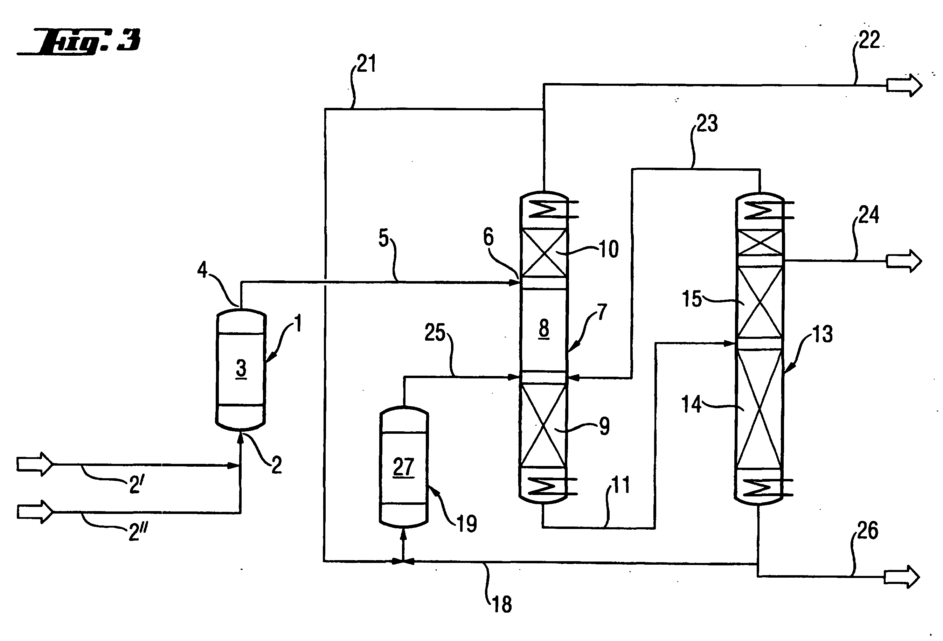

Eine weitere bevorzugte Ausführungsform der erfindungsgemäßen Vorrichtung ist in Figur 3 wiedergegeben. Diese erfindungsgemäße Vorrichtung unterscheidet sich von der erfindungsgemäßen Vorrichtung in Figur 2 darin, dass die Leitung **23** zur zumindest teilweisen Abführung des Destillats der Destillationskolonne **13** in die Reaktiv-Destillationskolonne **7** mit dieser verbunden ist. Die Leitung **23** wird hierbei vorzugsweise unterhalb der Katalysatorzone **8** und oberhalb der unteren Rektifikationszone **9** oder im oberen Drittel der unteren Rektifikationszone **9** in die Reaktiv-Destillationskolonne **7** geführt.

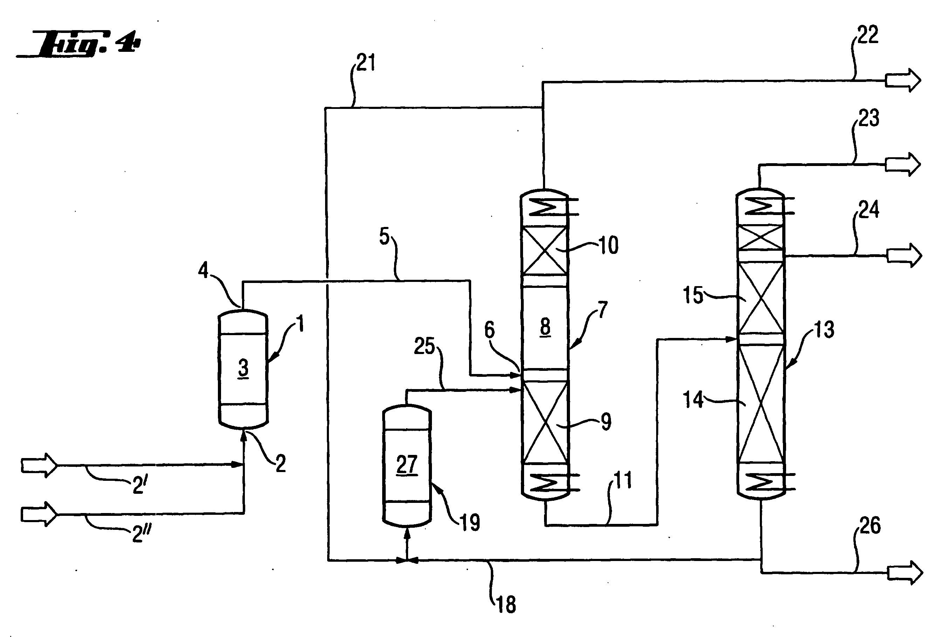

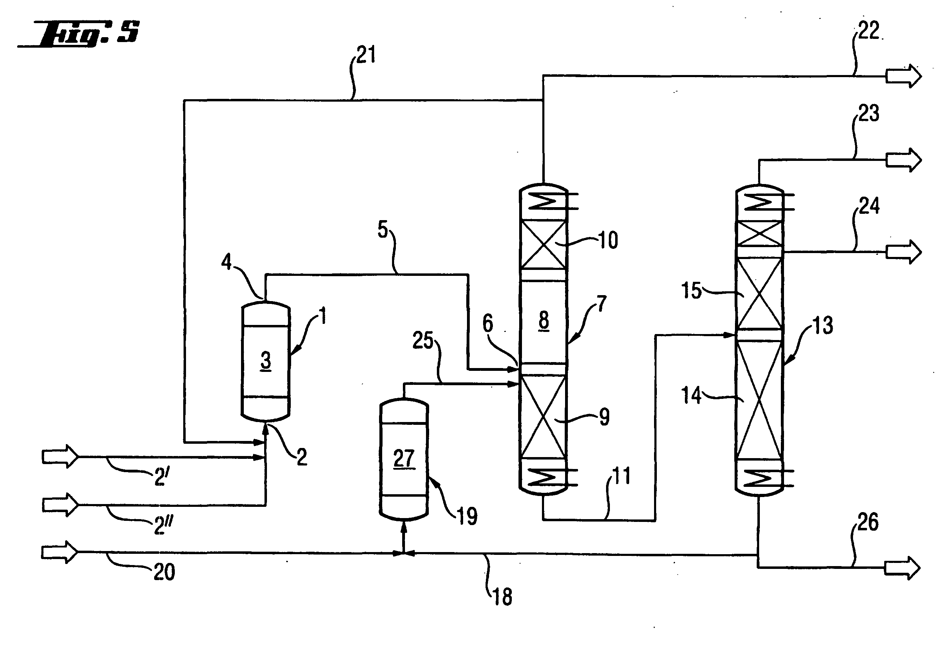

Weitere für die Hydrolyse höhersiedender Carbonsäureester mit erhöhtem Wasseranteil im leichtsiedenden, meist ternären Azeotrop bevorzugte Ausführungsformen sind in Figur 4 und 5 wiedergegeben. Diese erfindungsgemäße Vorrichtungen unterscheiden sich von der erfindungsgemäßen Vorrichtung in Figur 2 und 1 darin, dass das Produkt des ersten Vorreaktors **1** bevorzugt unterhalb des Reaktionsteils **8** und oberhalb der Rektifikationszone **9** oder im oberen Drittel der unteren Rektifikationszone **9** in die Reaktiv-Destillationskolonne **7** eingespeist wird. Das Produkt des zweiten Vorreaktors **19** wird unterhalb der Reaktionszone **8,** vorzugsweise im oberen Drittel der unteren Rektifikationszone **9** eingespeist.

Mit dem erfindungsgemäßen Verfahren lassen sich bevorzugt die Carbonsäureester Methyl- und Ethylformiat, Methyl-, Ethyl, propyl-, iso-Butyl- und tert.-Butylacetat, Methyl- und Ethylpropionat sowie Methyl-, Ethyl- und Propylbutyrat, insbesondere Methylacetat hydrolysieren.

Ein erfindungsgemäßes Verfahren sowie eine erfindungsgemäße Vorrichtung zur Hydrolyse eines Carbonsäureesters soll nachfolgend anhand der sauer katalysierten Hydrolyse von Methylacetat beispielhaft beschrieben werden. Ein Fluidstrom enthaltend das Azeotrop aus Methylacetat und Methanol, entsprechend einem molaren Verhältnis von 1,94:1, aus einer Polyvinylalkoholproduktion wird mit bezüglich Methylacetat wenigstens aequimolaren Menge Wasser, vorzugsweise einem 4- bis 7-fachen molaren Überschuss an Wasser vermischt. Als Wasser wird Heißwasser verwendet, das das Gemisch auf eine Temperatur zwischen 50 und 80 °C erwärmt. Das Gemisch wird in den vorzugsweise vertikal angeordneten ersten Vorreaktor 1 geleitet.

Der erste Vorreaktor 1 ist mit einem kationischen Ionentauscher als saurer Feststoffkatalysator bepackt. Der Katalysator hat vorzugsweise eine Korngröße zwischen ungefähr 0,35 bis 3 mm. Ein solches Katalysatormaterial ist beispielsweise unter dem Namen Amberlyst 15® von der Firma Rohm & Haas erhältlich. Alternative Katalysatoren sind beispielsweise Zeolithe, Aluminiumoxid, Siliziumoxid etc.

Das Gemisch von Methylacetat und Wasser durchströmt den ersten Vorreaktor **1** und wird dabei mit dem Katalysatormaterial in Kontakt gebracht. Dabei erfolgt eine teilweise Hydrolyse des Methylacetats. Die Umsetzung des Methylacetats im ersten Vorreaktor **1** beträgt zwischen 20 und 100%, vorzugsweise zwischen 60 und 100% des Gleichgewichtsumsatzes. Das entstehende Reaktionsgemisch wird über die Leitung **5** vorzugsweise oberhalb der Katalysatorzone **8** und unterhalb der Rektifikationszone **10** der Reaktiv-Destillationskolonne **7** eingeleitet.

Die Reaktiv-Destillationskolonne **7** wird vorzugsweise so betrieben, dass aus der Bodenfraktion die beiden Hydrolyseprodukte Methanol und Essigsäure mit dem nicht umgesetzten Wasser abgenommen werden. Das verbleibende, leichtflüchtige Methylacetat wird in der unteren Rektifikationszone **9** von den Reaktionsprodukten abgetrennt und destillativ in der Katalysatorzone **8** zur Hydrolyse aufkonzentriert. Die gebildeten Reaktionsprodukte Methanol und Essigsäure werden dabei permanent destillativ aus der Katalysatorzone **8** in die untere Rektifikationszone **9** bzw. in die Bodenfraktion, Leitung **11,** ausgeschleust.

Das in der Bodenfraktion der Reaktiv-Destillationskolonne **7** anfallende Gemisch aus Essigsäure, Wasser und Methanol wird in der nachgeschalteten Destillationskolonne **13** weiter getrennt, wobei Methanol am Kolonnenkopf oder an der oberen Destillationszone **15** abgezogen wird und ein Gemisch aus Essigsäure und Wasser in der Bodenfraktion der Destillationskolonne **13** anfällt. Diese wässrige Essigsäure wird über die Leitung **26** abgeleitet und zumindest teilweise über die Leitung **18** in den zweiten Vorreaktor **19** eingespeist. Alternativ kann die recyclierende Menge an wässriger Carbonsäure mittels eines Seitenabzugs der unteren Rektifikationszone **14,** vorzugsweise deren unteren Hälfte, entnommen werden.

Der Leitung **18** wird vor Eintritt in den zweiten Vorreaktor **19** über die Leitung **20** weiteres Methylacetat zugeführt. In einer weiteren bevorzugten Ausführungsform wird über die Leitung **20** ein Methylacetatstrom eingespeist, der bevorzugt 50 bis 100%, besonders bevorzugt 90 bis 100% Methylacetat enthält. Dieser Methylacetatstrom wird beispielsweise aus bereits weiter aufgereinigtem Methylacetat, das beispielsweise bei der Polyvinylalkoholproduktion anfällt, gespeist. Die wässrige Essigsäure aus der Bodenfraktion der Destillationskolonne **13** weist eine Temperatur höher als 30°C, bevorzugt 50 bis 110°C, besonders bevorzugt 100 bis 105°C auf. Durch die Mischung mit dem Methylacetat wird der Einlassstrom des zweiten Vorreaktors **19** bereits auf Temperaturen von 25 bis 100°C, bevorzugt auf 50 bis 90°C, besonders bevorzugt auf 70 bis 90°C erwärmt. Das Mischen der Zuflüsse und die Reaktion im zweiten Vorreaktor erfolgen aufgrund des hohen Dampfdrucks von Methylacetat vorzugsweise unter Überdruck.

Der zweite Vorreaktor **19** weist vorzugsweise einen sauren Feststoffkatalysator auf. Der zweite Vorreaktor **19** wird vorzugsweise von unten nach oben durchströmt und bei einer bevorzugten Temperatur von 50 bis 120°C, besonders bevorzugt von 70 bis 90°C betrieben. Die Umsetzung des Methylacetats beträgt zwischen 20 und 100%, vorzugsweise zwischen 60 und 100% des Gleichgewichtsumsatzes. Der Auslass des zweiten Vorreaktors **19** ist mittels der Leitung **25** mit der Reaktiv-Destillationskolonne **7** verbunden. Die Leitung **25** wird hierbei vorzugsweise unterhalb der Katalysatorzone **8** und oberhalb der unteren Rektifikationszone **9** oder im oberen Drittel der unteren Rektifikationszone **9** in die Reaktiv-Destillationskolonne **7** geführt.

In einer weiteren bevorzugten erfindungsgemäßen Ausführungsform wird die Leitung **21** Destillat der Reaktiv-Destillationskolonne **7** führend, welches gegebenenfalls nicht umgesetztes Methylacetat enthält, der Leitung **18** zugeführt. Die Leitung **20** entfällt dementsprechend. Diese Ausführungsform hat den Vorteil, dass nicht umgesetztes Methylacetat der Hydrolyse im zweiten Vorreaktor **19** und gegebenenfalls der Reaktiv-Destillationskolonne **7** zugeführt wird.

Die Temperatur im ersten Vorreaktor **1,** zweiten Vorreaktor **19** und in der Reaktiv-Destillationskolonne **7** wird in Abhängigkeit vom Druck festgelegt, wobei ein bestimmter Überdruck eine höhere Temperatur und somit eine höhere Reaktionsgeschwindigkeit und eine günstigere Gleichgewichtslage erlaubt. Vorzugsweise wird die Vorrichtung oder Teile der Vorrichtung bei Überdrücken von 1 bis 6 bar, besonders bevorzugt 1,5 bis 3 bar betrieben.

Wird abweichend zur Auslegung einer Anlage gemäß dem Stand der Technik ein Prozesszufluss mit einem molaren Verhältnis von Methylacetat zu Methanol 1,94:1 entsprechend dem Azeotrop in den Prozess eingeführt, schmälert dies bedingt durch die Verschiebung der Gleichgewichtsreaktion zu Lasten der Produkte die maximal verfügbare Kapazität einer Anlage gemäß dem Stand der Technik. Durch die erhöhte Rückführung von methylacetatreichem Destillat der Reaktiv-Destillationskolonne **7** auf den ersten Vorreaktor **1** und der weitestgehend fixierten Geometrie der Katalysatorzone **8** der Reaktiv-Destillationskolonne **7** ist der Umfang einer Kapazitätssteigerung unter Verwendung der bestehenden Struktur beschränkt.

Ein Azeotrop aus Methylacetat und Methanol wird nicht als Zufluss für den zweiten Vorreaktor **19** bevorzugt, da der so eingespeiste Alkoholanteil die Gleichgewichtsreaktion der sauren Hydrolyse zu ungunsten der Produkte verschiebt. Wie bereits erwähnt, ist das molare Verhältnis Methylacetat zu Methanol im Azeotrop 1,94:1. In der stark methylacetathaltigen Kopffraktion sowie im Destillat der Reaktiv-Destillationskolonne **7** liegt das Verhältnis etwa bei 4:1 oder höher, was erheblich günstiger für die Umsetzung ist. Es ist daher besonders vorteilhaft, das Destillat der Reaktiv-Destillationskolonne **7** in den zweiten Vorreaktor **19** einzuleiten, statt es dem Zulauf des ersten Vorreaktors **1** einzuspeisen. Eine Abnahme des Destillats aus der oberen Rektifikationszone **10** der Reaktiv-Destillationskolonne **7** begünstigt ferner durch den geringeren Gehalt an auszuschleusenden Leichtsiedern und den zur Katalysatorzone **8** hin anwachsenden Wasseranteil die Umsetzung. Ein Mengenverhältnis von etwa 1:1 eignet sich dabei bereits, das zugeführte Methylacetat mit einer Essigdünnsäure mit ca. 35 Gew.-% zu etwa 33 % umzusetzen. Dies bedeutet, dass über die zugeführte Destillatmenge, Leitung **21,** der Reaktiv-Destillationskolonne **7** der Methylacetat-Umsatz optimiert werden kann. Dies gilt ebenso für den recyclierten wässrigen Carbonsäurestrom, Leitung **18.** Ein erhöhter Einlassstrom an Azeotrop aus Methylacetat und Methanol auf den ersten Vorreaktor **1** ersetzt dabei die wegfallende Destillatmenge, Leitung **21,** der Reaktiv-Destillationskolonne **7** und erlaubt somit die Kapazitätssteigerung.

Die hohe Ablauftemperatur der wässrigen Essigsäure am Abtriebteil der Destillationskolonne **13** von vorzugsweise 100 bis 105°C ermöglicht eine Mischungstemperatur mit dem Methylacetat in der Größenordnung 70 bis 90 °C, was die Hydrolyse des zugeführten Methylacetats im zweiten Vorreaktor **19** begünstigt. Dabei wird eine Prozessführung unter leichtem Überdruck von 1,5 bis 3 bar besonders bevorzugt. Der Betrieb unter Überdruck kann durch eine geeignete Rohrleitungsführung einfach Rechnung getragen werden.

Das Recyclieren von Dünnsäure auf einen zweiten Vorreaktor **19** und das Einspeisen in die Reaktiv-Destillation **7** führt zu einer deutlich erhöhten fluiddynamischen Belastung der Abtriebteile (**9, 10**) der Reaktiv-Destillationskolonne **7** und der gegebenenfalls nachgeschalteten Destillationskolonne **13.**

Die Kapazität einer Anlage gemäß dem Stand der Technik lässt sich durch Einführung eines zweiten Vorreaktors **19** und die Rückführung der wässrigen Carbonsäure auf zusätzlich 80 bis 100% abschätzen, wobei sich der spezifische Heizdampfbedarf der Anlage nur geringfügig erhöht.

Die installationstechnische und prozesstechnische Einbindung des zweiten Vorreaktors **19** gestaltet sich einfach. Die erfindungsgemäße bevorzugte Ausführungsform erlaubt es ebenfalls, höher konzentrierte wässrige Essigsäure zu produzieren, indem der Wasserzufluss auf den ersten Vorreaktor **1** nicht proportional zur Methylacetatmenge gesteigert wird.

In den nachfolgenden Versuchsbeispielen wurde als Katalysator ein handelsüblicher kationischer Feststoffkatalysator eingesetzt. In der Reaktiv-Destillationskolonne **7** wurde der Katalysator in eine strukturierte Katalysatorpackung eingebracht.

**Beispiel 1 (Vergleichsbeispiel):**

Es wurde eine Kombination aus dem Vorreaktor **1** mit Reaktiv-Destillationskolonne **7** eingesetzt. Es erfolgte keine Rückführung von Destillat von der Reaktiv-Destillationskolonne **7** auf den Vorreaktor **1** mittels Leitung **21.** Der zu hydrolysierende Methylacetatstrom wies dabei annähernd die azeotrope Zusammensetzung von 81 Gew.-% Methylacetat und 19 Gew.-% Methanol auf. Die Bodenfraktion der Reaktiv-Destillationskolonne **7** enthält Methanol, Essigsäure, Wasser und Spuren von Methylacetat. Dieses Gemisch wurde in der Destillationskolonne **13** in einen Spuren von Methylacetat enthaltenden Methanolstrom und in ein Essigsäure/Wasser-Gemisch aufgetrennt.

Reaktiv-Destillationskolonne **7**:

| | |
|---|---|
| Durchmesser: | 1100 mm |
| Oberer Rektifikationszone 1**0**: | 5 theoretische Stufen |
| Katalysatorzone **8**: | 3 theoretische Stufen |
| Untere Rektifikationszone **9**: | 8 theoretische Stufen |

Einsatzströme:

| | |
|---|---|
| Azeotrop, Leitung **2'**: | 700 kg/h |
| Wasser, Leitung **2''**: | 900 kg/h |

Produktströme:

| | |
|---|---|
| Kopffraktion (Purge), Leitung **22**, der Reaktiv-Destillationskolonne **7**: | 26 kg/h |
| Bodenfraktion, Leitung **11**, der Reaktiv-Destillationskolonne **7**: | 1574 kg/h |

Versuchsbedingungen:

| | |
|---|---|
| Kopfdruck Reaktiv-Destillationskolonne **7**: | 26 mbarü |
| Einspeisepunkt Reaktiv-Destillationskolonne **7**: | oberhalb der Katalysatorzone 8 |
| Heizleistung Reaktiv-Destillationskolonne **7**: | 700 kW |
| Heizleistung Destillationskolonne **13**: | 490 kW |
| Eintrittstemperatur Vorreaktor **1**: | 58°C |

<div style="text-align:center"><u>Resultat:</u></div>

| | |
|---|---|
| Methylacetatumsetzung **1**: | 41,8 % |
| Methylacetatumsetzung Gesamt: | 98,9 % |
| | bei Vernachlässigung des Purge, Leitung **22**) |

<u>Zusammensetzung Bodenfraktion, Leitung **11**, der Reaktiv-Destillationskolonne **7**:</u>

| | |
|---|---|
| Methylacetat: | 0,4 Gew.-% |
| Methanol: | 22,7 Gew.-% |
| Wasser: | 49,6 Gew.-% |
| Essigsäure: | 27,3 Gew.-% |

<u>Zusammensetzung Kopffraktion, Leitung 23, der Destillationskolonne **13**</u>:

| | |
|---|---|
| Methylacetat: | 2,0 Gew.-% |
| Methanol: | 98,0 Gew.-% |

<u>Zusammensetzung Bodenfraktion, Leitung 26, der Destillationskolonne **13**</u>:

| | |
|---|---|
| Wasser: | 64,3 Gew.-% |
| Essigsäure: | 35,7 Gew.-% |

**Beispiel 2 (Vergleichsbeispiel - Prozess mit Rückführung):**

Es wurde eine Kombination aus dem Vorreaktor **1** mit Reaktiv-Destillationskolonne **7** eingesetzt. Es erfolgte eine Rückführung von Destillat von der Reaktiv-Destillationskolonne **7** auf den Vorreaktor **1** mittels Leitung **21**. Der zu hydrolysierende Methylacetatstrom wies dabei annähernd die azeotrope Zusammensetzung von 81 Gew.-% Methylacetat und 19 Gew.-% Methanol auf. Die Bodenfraktion der Reaktiv-Destillationskolonne **7** enthält Methanol, Essigsäure, Wasser und Spuren von Methylacetat. Dieses Gemisch wurde in der Destillationskolonne **13** in einen Spuren von Methylacetat enthaltenden Methanolstrom und in ein Essigsäure/Wasser-Gemisch aufgetrennt.

<u>Reaktiv-Destillationskolonne **7**:</u>

| | |
|---|---|
| Durchmesser: | 1100 mm |
| Obere Rektifikationszone **10**: | 5 theoretische Stufen |
| Katalysatorzone **8**: | 3 theoretische Stufen |
| Untere Rektifikationszone **9**: | 8 theoretische Stufen |

<u>Einsatzströme:</u>

| | |
|---|---|
| Azeotrop, Leitung **2'**: | 685 kg/h |
| Wasser, Leitung **2"**: | 930 kg/h |

<u>Produktströme:</u>

| | |
|---|---|
| Kopffraktion (Purge), Leitung **22**, der Reaktiv-Destillationskolonne **7**: | 15 kg/h |
| Bodenfraktion, Leitung **11**, der Reaktiv-Destillationskolonne **7**: | 1600 kg/h |

<u>Versuchsbedingungen:</u>

| | |
|---|---|
| Kopfdruck Reaktiv-Destillationskolonne **7**: | 29 mbarü |
| Einspeisepunkt Reaktiv-Destillationskolonne **7:** | oberhalb der Katalysatorzone **8** |

<div style="text-align:center">9</div>

Tabelle fortgesetzt

| | |
|---|---|
| Destillatrückführung, Leitung **21**, auf Vorreaktor **1**: | 1985 kg/h |
| Heizleistung Reaktiv-Destillationskolonne **7**: | 490 kW |
| Heizleistung Destillationskolonne **13**: | 490 kW |
| Eintrittstemperatur Vorreaktor **1**: | 58°C |

Resultat:

| | |
|---|---|
| Methylacetatumsetzung | 99,1 % |
| | (bei Vernachlässigung des Purge, Leitung **22**) |

Zusammensetzung Bodenfraktion, Leitung **11**, der Reaktiv-Destillationskolonne **7**:

| | |
|---|---|
| Methylacetat: | 0,3 Gew.-% |
| Methanol: | 22,7 Gew.-% |
| Wasser: | 50,0 Gew.-% |
| Essigsäure: | 27,4 Gew.-% |

Zusammensetzung Kopffraktion, Leitung **23**, der Destillationskolonne **13**:

| | |
|---|---|
| Methylacetat: | 1,5 Gew.-% |
| Methanol: | 98,5 Gew.-% |

Zusammensetzung Bodenfraktion, Leitung **26**, der Destillationskolonne **13**:

| | |
|---|---|
| Wasser: | 69,6 Gew.-% |
| Essigsäure: | 35,4 Gew.-% |

**Beispiel 3:**

Es wurde eine Kombination aus zwei Vorreaktoren **1** und **19** mit einer Reaktiv-Destillationskolonne **7** gemäß Figur 2 eingesetzt. Der zu hydrolysierende Methylacetatstrom wies dabei annähernd die azeotrope Zusammensetzung von 81 Gew.-% Methylacetat und 19 Gew.-% Methanol auf. Die Bodenfraktion der Reaktiv-Destillationskolonne **7** enthält Methanol, Essigsäure, Wasser und Spuren von Methylacetat. Dieses Gemisch wurde in der Destillationskolonne **13** in einen Spuren von Methylacetat enthaltenden Methanolstrom und in ein Essigsäure/Wasser-Gemisch aufgetrennt.

Reaktiv-Destillationskolonne **7**:

| | |
|---|---|
| Durchmesser: | 1100 mm |
| Obere Rektifikationszone **10**: | 5 theoretische Stufen |
| Katalysatorzone **8**: | 3 theoretische Stufen |
| Untere Rektifikationszone **9**: | 8 theoretische Stufen |

Einsatzströme:

| | |
|---|---|
| Azeotrop, Strom **2'**: | 1600 kg/h |
| Wasser, Strom **2''**: | 2200 kg/h |

Produktströme:

| | |
|---|---|
| Kopffraktion (Purge), Leitung **22**, der Reaktiv-Destillationskolonne **7**: | 30 kg/h |
| Bodenstrom, Leitung **11**, der Reaktiv-Destillationskolonne **7**: | 6170 kg/h |
| Destillatrückführung, Leitung **21**, auf zweiten Vorreaktor **19**: | 3000 kg/h |

Tabelle fortgesetzt

| | |
|---|---|
| Dünnsäure-Rückführung, Leitung **18**, auf zweiten Vorreaktor **19**: | 2400 kg/h |
| Kopffraktion, Leitung **23**, der Destillationskolonne **13**: | 870 kg/h |
| Bodenfraktion, Leitung **26**, Destillationskolonne **13**: | 2900 kg/h |

Versuchsbedingungen:

| | |
|---|---|
| Kopfdruck der Reaktiv-Destillationskolonne **7**: | 30 mbarü |
| Einlass **6** in Reaktiv-Destillationskolonne **7**: | oberhalb der Katalysatorzone **8** |
| Einlass der Leitung **25** in Reaktiv-Destillationskolonne **7**: | unterhalb der Katalysatorzone **8** |
| Heizleistung Reaktiv-Destillationskolonne **7**: | 1200 kW |
| Heizleistung Destillationskolonne **13**: | 1350 kW |
| Eintrittstemperatur Vorreaktor **1**: | 62 °C |
| Eintrittstemperatur Vorreaktor **19**: | 88 °C |

Resultat:

| | |
|---|---|
| Methylacetatumsetzung: | 98,8 % |
| | bei Vernachlässigung des Purge, Leitung **22**) |

Zusammensetzung Bodenfraktion, Leitung **11**, der Reaktiv-Destillationskolonne **7**:

| | |
|---|---|
| Methylacetat: | 0,3 Gew.-% |
| Methanol: | 14,0 Gew.-% |
| Wasser: | 55,7 Gew.-% |
| Essigsäure: | 30,0 Gew.-% |

Zusammensetzung Kopffraktion, Leitung **23**, der Destillationskolonne **13**:

| | |
|---|---|
| Methylacetat: | 1,8 Gew.-% |
| Methanol: | 98,2 Gew.-% |

Zusammensetzung Bodenfraktion, Leitung **11**, der Destillationskolonne **13**:

| | |
|---|---|
| Wasser: | 65,0 Gew.-% |
| Essigsäure: | 35,0 Gew.-% |

**Beispiel 4:**

Es wurde eine Kombination von zwei Vorreaktoren **1** und **19** mit einer Reaktiv-Destillationskolonne **7** gemäß Figur 3 eingesetzt. Der Zufluss an Methylacetat wurde erhöht und der Wasserüberschuss reduziert. Der zu hydrolysierende Methylacetatstrom wies dabei annähernd die azeotrope Zusammensetzung von 81 Gew.-% Methylacetat und 19 Gew.-% Methanol auf. Die Bodenfraktion der Reaktiv-Destillationskolonne **7** enthält Methanol, Essigsäure, Wasser und Spuren von Methylacetat. Dieses Gemisch wurde in einer Destillationskolonne **13** in einen Spuren von Methylacetat enthaltenden Methanolstrom sowie in ein Gemisch aus Essigsäure und Wasser aufgetrennt.

Reaktiv-Destillationskolonne **7**:

| | |
|---|---|
| Durchmesser: | 1100 mm |
| Obere Rektifikationszone **10**: | 5 theoretische Stufen |
| Katalysatorzone **8**: | 3 theoretische Stufen |
| Untere Rektifikationszone **9**: | 8 theoretische Stufen |

Einsatzströme:

Azeotrop, Leitung **2'**:     1700 kg/h

Wasser, Leitung **2''**:     1700 kg/h

Produktströme:

| | |
|---|---|
| Kopffraktion(Purge), Leitung **22**, der Reaktiv-Destillationskolonne **7**: | 35 kg/h |
| Bodenfraktion, Leitung **11**, der Reaktiv-Destillationskolonne **7**: | 6865 kg/h |
| Destillatrückführung, Leitung **21**, auf zweiten Vorreaktor **19**: | 3500 kg/h |
| Dünnsäure-Rückführung,Leitung **18**, auf zweiten Vorreaktor **19**: | 3500 kg/h |
| Destillat, Leitung **23**, der Destillationskolonne **13**: | 930 kg/h |
| Bodenfraktion, Leitung **26**, Destillationskolonne **13**: | 2435 kg/h |

Versuchsbedingungen:

| | |
|---|---|
| Kopfdruck der Reaktiv-Destillationskolonne **7**: | 37 mbar |
| Einlass **6** in die Reaktiv-Destillationskolonne **7**: | oberhalb der Katalysatorzone **8** (bei Vernachlässigung des Purge, Leitung **22**) |
| Einlass der Leitung **25** in Reaktiv-Destillationskolonne **7**: | unterhalb der Katalysatorzone **8** |
| Heizleistung Reaktiv-Destillationskolonne **7**: | 1300 kW |
| Heizleistung Destillationskolonne **13**: | 1300 kW |
| Eintrittstemperatur Vorreaktor **1**: | 65°C |
| Eintrittstemperatur Vorreaktor **19**: | 77 °C |

Resultat:

Methylacetatumsetzung:     98,2 %

Zusammensetzung Bodenfraktion, Leitung **11**, der Reaktiv-Destillationskolonne **7**:

| | |
|---|---|
| Methylacetat: | 0,4 Gew.-% |
| Methanol: | 13,3 Gew.-% |
| Wasser: | 48,4 Gew.-% |
| Essigsäure: | 37,9 Gew.-% |

Zusammensetzung Kopffraktion, Leitung **23**, der Destillationskolonne **13**:

| | |
|---|---|
| Methylacetat: | 2,7 Gew.-% |
| Methanol: | 97,3 Gew.-% |

Zusammensetzung Bodenfraktion, Leitung **26**, der Destillationskolonne **13**:

| | |
|---|---|
| Wasser: | 56,1 Gew.-% |
| Essigsäure: | 43,9 Gew.-% |

**Beispiel 5:**

Als weiteres erfindungsgemäßes Beispiel wird die Hydrolyse von Isobutylacetat in Isobutanol und Essigsäure mittels Prozesssimulation betrachtet. Isobutylacetat wurde gewählt, da dessen Normalsiedepunkt in der Gruppe Isobutylacetat, Isobutanol, Essigsäure und Wasser am höchsten liegt. Die Siedepunkte der beiden Reaktionsprodukte Isobutanol und Essigsäure liegen über dem des Reaktionspartners Wasser.

<u>Normalsiedepunkte:</u>

| | |
|---|---|
| Isobutylacetat (iBuAc): | 117,2 °C |
| Wasser ($H_2O$) : | 100,0 °C |
| Isobutanol (iBuOH): | 107,9 °C |
| Essigsäure (ES): | 117,9 °C |

<u>Binäre Azeotrope:</u>

77,7 Gew.-% Isobutylacetat, 22,3 Gew.-% Wasser; Normalsiedepunkt: 88,4 °C
44,9 Gew.-% Isobutylacetat, 55,1 Gew.-% Isobutanol; Normalsiedepunkt: 105,1 °C
66,8 Gew.-% Isobutanol, 33,2 Gew.-% Wasser; Normalsiedepunkt: 89,9 °C

<u>Ternäres Azeotrop</u>

46,5 Gew.-% Isobutylacetat, 30,4 Gew.-% Wasser, 23,1 Gew.-% Isobutanol; Normalsiedepunkt: 86,8 °C
52,3 Gew.-% Isobutylacetat, 21,3 Gew.-% Wasser, 26, 4 Gew.-% Isobutanol; Normalsiedepunkt: 86,6 °C

Der Prozess wurde aufbauend auf Gleichgewichtsberechnungen seitens des Stoffübergangs und der chemischen Umsetzung durchgeführt. Die Dimerisierung der Carbonsäure wurde gemäß dem Ansatz von Hayden O'Connel berücksichtigt. Für die Beschreibung des chemischen Gleichgewichts wurde die Gleichgewichtskonstante gemäß Formel (1)

$$K_x = (x(iBuOH)*x(ES))/(x(iBuAc)*x(H_2O)) = 0,05 \ mol^2/mol^2 \qquad (1)$$

verwendet.

Die gewählte Prozessführung ist in Figur 5 dargestellt. Sie basiert auf der Figur 1, jedoch wurden die Zulaufstellen auf die Reaktiv-Destillationskolonne 7 den Siedebedingungen des Stoffsystems angepasst.

<u>Reaktiv-Destillationskolonne 7</u>:

| | |
|---|---|
| Obere Rektifikationszone **10**: | Stufe 1 bis 4; 4 theoretische Stufen |
| Katalysatorzone **8**: | Stufe 5 bis 7; 3 theoretische Stufen |
| untere Rektifikationszone **9**: | Stufe 8 bis 23; 16 theoretische Stufen |
| Rücklaufverhältnis: Zulauf von ersten Vorreaktor **1**: | 1,12 oberhalb Stufe 8; unterhalb der Katalysatorzone **8** |
| Zulauf von zweiten Vorreaktor **19**: | oberhalb Stufe 11; im oberen Drittel der unteren Rektifikationszone **9** |
| Erster Vorreaktor **1**: | Temperatur: 100°C |
| | Druck: 1 barü |
| | Chemisches Gleichgewicht |
| Zweiter Vorreaktor **19**: | Temperatur: 100 °C |
| | Druck: 1 barü |
| | Chemisches Gleichgewicht |
| Destillationskolonne **13**: | 28 Trennstufen |
| | Rücklaufverhältnis: 2,5 |
| Verschaltung: | Leitung **21** entspringt Leitung **22** |
| | Leitung **18** entspringt Leitung **26** |

Einsatz- und Produktströme:

| Leitung | 2' | 2" | 20 | 5 | 11 | 18 | 21 | 22 | 23 | 25 | 26 |
|---|---|---|---|---|---|---|---|---|---|---|---|
| Fluss (kg/h) | 1000 | 1980 | 350 | 8914 | 8264 | 5000 | 5934 | 66 | 1400 | 5350 | 1864 |
| $H_2O$ (Gew.-%) | | 100 | | 37,0 | 60,6 | 64,8 | 24,7 | 24,7 | 39,9 | 60,4 | 64,8 |
| iBuAc (Gew.-%) | 97,0 | | 97,0 | 33,1 | 0,01 | | 48,7 | 48,7 | 0,09 | 5,1 | |
| ES (Gew.-%) | | | | 5,3 | 29,2 | 35,2 | | | | 33,5 | 35,2 |
| iBuOH (Gew.-%) | 3,0 | | 3,0 | 24,6 | 10,1 | | 26,6 | 26,6 | 60,1 | 1,0 | |

Der Vergleich der Simulation der Hydrolyse von Isobutylacetat mittels einer Verschaltung des Vorreaktors **1** mit der Reaktivdestillationskolonne **7** und der Rückführung gleicher Destillatmengen mittels Leitung **21** bei gleicher Heizleistung und gleichem Wasserüberschuss, Leitung **2"**, zeigt, dass nur der Prozesszulauf **2'** von 1000 kg/h umgesetzt werden kann. Die erfindungsgemäße Einbindung des zweiten Vorreaktors **19** erlaubt somit den Prozesszufluss von 1350 kg/h zu hydrolysieren. Dies entspricht einer Kapazitätssteigerung von 35%.

**Patentansprüche**

1. Verfahren zur sauer katalysierten Hydrolyse eines Carbonsäureesters in die entsprechende Carbonsäure und den entsprechenden Alkohol in Gegenwart von Wasser, wobei ein Einlassstrom enthaltend den Carbonsäureester mit Wasser vermischt in einen ersten Vorreaktor aufweisend einen Hydrolysekatalysator geleitet wird, wodurch der Carbonsäureester zumindest teilweise in die Hydrolyseprodukte gespalten wird, das Reaktionsgemisch aus diesem ersten Vorreaktor abgezogen und wenigstens teilweise in eine Reaktiv-Destillationskolonne enthaltend einen Hydrolyse-Katalysator geleitet wird, wodurch der Carbonsäureesterstrom enthaltend Wasser weiter in Carbonsäure und Alkohol umgesetzt und zeitgleich wenigstens teilweise in die Komponenten getrennt wird, wobei die leichter flüchtigen Verbindungen und die den leichtsiedenden Carbonsäureester enthaltenden Mischungen zumindest teilweise aus der oberen Rektifikationszone der Reaktiv-Destillationskolonne und dem zugehörigen Kondensationssystem der Reaktiv-Destillationskolonne als Destillat abgezogen werden und die schwerer flüchtigen Verbindungen sich wenigstens teilweise als Bodenfraktion sammeln, die in eine weitere Destillationskolonne geleitet wird, **dadurch gekennzeichnet, dass** die wässrige Carbonsäure aus der Bodenfraktion oder aus der unteren Rektifikationszone der Destillationskolonne mit weiterem Carbonsäureester vermischt einem zweiten Vorreaktor enthaltend einen Hydrolyse-Katalysator zugeführt wird, das Reaktionsgemisch aus diesem zweiten Vorreaktor abgezogen und wenigstens teilweise in die Reaktiv-Destillationskolonne geleitet wird.

2. Verfahren nach Anspruch 1, **dadurch gekennzeichnet, dass** die wässrige Carbonsäure aus der Bodenfraktion oder aus der unteren Rektifikationszone der Destillationskolonne mit einem separat zugeführten Carbonsäureesterstrom oder Carbonsäureester enthaltenden Strom vermischt und anschließend dem zweiten Vorreaktor zugeführt wird.

3. Verfahren nach mindestens einem der Ansprüche 1 oder 2, **dadurch gekennzeichnet, dass** das Destillat der Reaktiv-Destillationskolonne enthaltend noch nicht umgesetzten Carbonsäureester zumindest teilweise wieder dem ersten Vorreaktor zugeführt wird.

4. Verfahren nach mindestens einem der Ansprüche 1 bis 3, **dadurch gekennzeichnet, dass** die wässrige Carbonsäure aus der Bodenfraktion oder aus der unteren Rektifikationszone der Destillationskolonne mit dem Destillat der Reaktiv-Destillationskolonne enthaltend noch nicht umgesetzten Carbonsäureester vermischt und anschließend dem zweiten Vorreaktor zugeführt wird.

5. Verfahren nach mindestens einem der Ansprüche 1 bis 4, **dadurch gekennzeichnet, dass** der wässrige, heiße Carbonsäurestrom aus der Bodenfraktion oder aus der unteren Rektifikationszone der Destillationskolonne zum Erwärmen des mit diesem zu vermischenden Stroms enthaltend Carbonsäureester genutzt wird.

6. Verfahren nach mindestens einem der Ansprüche 1 bis 5, **dadurch gekennzeichnet, dass** das Reaktionsprodukt aus dem zweiten Vorreaktor unterhalb der Reaktionszone und oberhalb der unteren Rektifikationszone oder im oberen Drittel der unteren Rektifikationszone der Reaktiv-Destillationskolonne eingespeist wird.

7. Verfahren nach mindestens einem der Ansprüche 1 bis 6, **dadurch gekennzeichnet, dass** der Carbonsäureester ausgewählt wird aus der Gruppe enthaltend Methyl- und Ethylformiat, Methyl-, Ethyl-, Propyl-, iso-Butyl- und *tert.*-Butylacetat, Methyl- und Ethylpropionat sowie Methyl-, Ethyl- und Propylbutyrat.

8. Verfahren nach mindestens einem der Ansprüche 1 bis 7, **dadurch gekennzeichnet, dass** der Einlassstrom auf den ersten Vorreaktor das Azeotrop des Carbonsäureesters mit dem entsprechenden Alkohol enthält.

9. Verfahren nach mindestens einem der Ansprüche 1 bis 8, **dadurch gekennzeichnet, dass** die Leichtsieder dem Destillat der Reaktiv-Destillationskolonne zumindest teilweise entnommen werden.

10. Verfahren nach mindestens einem der Ansprüche 1 bis 9, **dadurch gekennzeichnet, dass** das Verfahren kontinuierlich betrieben wird.

11. Vorrichtung zur sauer katalysierten Hydrolyse eines Carbonsäureesters in die entsprechende Carbonsäure und den entsprechenden Alkohol in Gegenwart von Wasser nach einem Verfahren gemäß mindestens einem der Ansprüche 1 bis 10, enthaltend

   a) einen ersten Vorreaktor **1** enthaltend einen Hydrolyse-Katalysator mit wenigstens einer Einlassleitung **2** zur Einspeisung eines Fluidstroms enthaltend den Carbonsäureester, Leitung **2'**, und Wasser, Leitung **2''**, sowie wenigstens einem Auslass **4**, zur Abführung des Reaktionsgemisches,
   b) wenigstens eine Heizvorrichtung zum Heizen des Einlassstroms, Leitung **2**, des ersten Vorreaktors **1**, oder von beiden,
   c) einen zweiten Vorreaktor **19** enthaltend einen Hydrolyse-Katalysator mit mindestens einem Einlass für einen Fluidstrom enthaltend zumindest teilweise die wässrige Carbonsäure aus der Bodenfraktion oder einer Fraktion aus der unteren Rektifikationszone **14** der Destillationskolonne **13**, Leitung **18**, weiterhin vermischt mit einem Carbonsäureesterstrom, Leitung **20**, und mindestens einem Auslass, Leitung **25**,
   d) eine Reaktiv-Destillationskolonne **7** enthaltend eine Katalysatorzone **8** aufweisend einen Hydrolyse-Katalysator mit einem Einlass **6** verbunden mit dem ersten Vorreaktor **1**, einem Einlass verbunden mit dem zweiten Vorreaktor, Leitung 25, wobei die Katalysatorzone **8** zwischen einer unteren Rektifikationszone **9** und einer oberen Rektifikationszone **10** angeordnet ist,
   e) am Destillationskopf der Reaktiv-Destillationskolonne angeschlossene Leitung **22** zur Abführung der Kopffraktion oder als Purge,
   f) mit der die Kopffraktion der Reaktiv-Destillationskolonne **7** führende Leitung **22** oder mit der oberen Rektifikationszone 10 oder dem Kondensationssystems der Reaktiv-Destillationskolonne **7** verbundene Leitung **21** zur Abführung von Destillat,
   g) am Destillationsfuß der Reaktiv-Destillationskolonne **7** angeschlossene Leitung **11** zum Abziehen der Bodenfraktion,
   h) eine Destillationskolonne **13**, wobei die Leitung **11** zum Abziehen der Bodenfraktion der Reaktiv-Destillationskolonne **7** den Einlass bildet, an deren Destillationsfuß, Leitung **26**, wässrige Carbonsäure abgezogen wird,
   i) Leitung **18**, führend eine Fraktion wässriger Carbonsäure entnommen der unteren Rektifikationszone **14** der Destillationskolonne **13** oder eine Teilmenge der Leitung **26**, wobei diese mit Carbonsäureester, Leitung **20**, vermischt den Zufluss auf den zweiten Vorreaktor **19** bildet.

12. Vorrichtung nach Anspruch 11, **dadurch gekennzeichnet, dass** die Leitung **18** zum Abziehen der wässrigen Carbonsäure aus der Destillationskolonne **13** vor Eintritt in den zweiten Vorreaktor **19** mit einer Leitung **20** führend einen weiteren Carbonsäureesterstrom verbunden wird.

13. Vorrichtung nach mindestens einem der Ansprüche 11 oder 12, **dadurch gekennzeichnet, dass** die den nicht umgesetzten Carbonsäureester führende Leitung **21** von der Reaktiv-Destillationskolonne **7** mit der Einlassleitung **2** oder einem der beiden Prozeßzuläufen, Leitungen **2'** und **2''**, des ersten Vorreaktors **1**, die diesen mit Carbonsäureester und Wasser speist, verbunden ist.

14. Vorrichtung nach Anspruch 13, **dadurch gekennzeichnet, dass** die Leitung **21** nicht umgesetzten Carbonsäureester der unteren Hälfte der oberen Rektifikationszone **10** der Reaktiv-Destillationskolonne **7** entnimmt.

15. Vorrichtung nach mindestens einem der Ansprüche 11 bis 14, **dadurch gekennzeichnet, dass** die den wässrigen Carbonsäurestrom führende Leitung **18** vor Eintritt in den zweiten Vorreaktor **19** mit der Leitung **21** verbunden ist, die zumindest teilweise die Kopffraktion oder eine Fraktion aus der oberen Rektifikationszone **10** oder des zugehörigen Kondensationssystems der Reaktiv-Destillationskolonne **7** führt.

16. Vorrichtung nach mindestens einem der Ansprüche 11 bis 15, **dadurch gekennzeichnet, dass** die Reaktiv-Destillationskolonne **7** einen Einlass **6** für den Zustrom aus dem ersten Vorreaktor **1** im oberen Drittel der Katalysatorzone **8** oder oberhalb der Katalysatorzone **8** und unterhalb der oberen Rektifikationszone **10** aufweist.

17. Vorrichtung nach mindestens einem der Ansprüche 11 bis 16, **dadurch gekennzeichnet, dass** die Reaktiv-Destillationskolonne **7** einen Einlass **6** für den Zustrom aus dem ersten Vorreaktor **1** unterhalb der Katalysatorzone **8** und oberhalb der unteren Rektifikationszone **9** oder im oberen Drittel der unteren Rektifikationszone **10** aufweist.

18. Vorrichtung nach mindestens einem der Ansprüche 11 bis 17, **dadurch gekennzeichnet, dass** die Reaktiv-Destillationskolonne **7** einen Einlass für die Leitung **25** vom zweiten Vorreaktor **19** unterhalb der Katalysatorzone **8** und oberhalb der unteren Rektifikationszone **9** oder im oberen Drittel der unteren Rektifikationszone **9** aufweist.

19. Vorrichtung nach mindestens einem der Ansprüche 11 bis 18, **dadurch gekennzeichnet, dass** die Hydrolyse-Katalysatoren im ersten Vorreaktor **1** oder zweiten Vorreaktor **19** unabhängig von einander Schüttungen eines festen Katalysators, in strukturierten Katalysatorpackungen fixiert oder in Rührwerken eingebracht oder aufgewirbelt sind.

20. Vorrichtung nach mindestens einem der Ansprüche 11 bis 19, **dadurch gekennzeichnet, dass** der Hydrolyse-Katalysator in der Reaktiv-Destillationskolonne **7** eine Schüttung eines festen Katalysators, in strukturierten Katalysatorpackung fixiert oder angeordnet auf den Böden sowie in den Ablaufschächten einer Bodenkolonne ist.

**Claims**

1. Process for acid-catalysed hydrolysis of a carboxylic ester to the corresponding carboxylic acid and the corresponding alcohol in the presence of water by passing an inlet stream comprising the carboxylic ester mixed with water into a first prereactor having a hydrolysis catalyst, which hydrolyses the carboxylic ester at least partly to the hydrolysis products, removing the reaction mixture from this first prereactor and passing it at least partly into a reactive distillation column comprising a hydrolysis catalyst, which converts the carboxylic ester stream comprising water further to carboxylic acid and alcohol and simultaneously at least partly separates it into the components, the mixtures comprising the less volatile compounds and the low-boiling carboxylic ester being removed at least partly as a distillate from the upper rectification zone of the reactive distillation column and the accompanying condensation system of the reactive distillation column and the less volatile compounds collecting at least partly as a bottom fraction which is passed into a further distillation column, **characterized in that** the aqueous carboxylic acid is fed from the bottom fraction or from the lower rectification zone on the distillation column, mixed with further carboxylic ester, to a second prereactor comprising a hydrolysis catalyst, the reaction mixture is removed from the second prereactor and passed at least partly into the reactive distillation column.

2. Process according to Claim 1, **characterized in that** the aqueous carboxylic acid from the bottom fraction or from the lower rectification zone of the distillation column is mixed with a separately fed carboxylic ester stream or carboxylic ester-containing stream and subsequently fed to the second prereactor.

3. Process according to at least one of Claims 1 and 2, **characterized in that** the distillate of the reactive distillation column comprising carboxylic ester which is yet to be converted is fed at least partly back to the first prereactor.

4. Process according to at least one of Claims 1 to 3, **characterized in that** the aqueous carboxylic acid from the bottom fraction or from the lower rectification zone of the distillation column is mixed with the distillate of the reactive distillation column comprising carboxylic ester which is yet to be converted and subsequently fed to the second prereactor.

5. Process according to at least one of Claims 1 to 4, **characterized in that** the aqueous, hot carboxylic acid stream from the bottom fraction or from the lower rectification zone of the distillation column is utilized for heating the stream comprising carboxylic ester to be mixed with it.

6. Process according to at least one of Claims 1 to 5, **characterized in that** the reaction product from the second prereactor is fed in below the reaction zone and above the lower rectification zone or in the upper third of the lower rectification zone of the reactive distillation column.

7. Process according to at least one of Claims 1 to 6, **characterized in that** the carboxylic ester is selected from the group consisting of methyl and ethyl formate, methyl, ethyl, propyl, isobutyl and tert-butyl acetate, methyl and ethyl propionate and methyl, ethyl and propyl butyrate.

8. Process according to at least one of Claims 1 to 7, **characterized in that** the inlet stream to the first prereactor comprises the azeotrope of the carboxylic ester with the corresponding alcohol.

9. Process according to at least one of Claims 1 to 8, **characterized in that** the low boilers are at least partly removed from the distillate of the reactive distillation column.

10. Process according to at least one of Claims 1 to 9, **characterized in that** it is operated continuously.

11. Apparatus for acid-catalysed hydrolysis of a carboxylic ester to the corresponding carboxylic acid and the corresponding alcohol in the presence of water by a process according to at least one of Claims 1 to 10, comprising

   a) a first prereactor **1** comprising a hydrolysis catalyst and having at least one inlet line **2** for feeding in a fluid stream comprising the carboxylic ester, line **2'**, and water, line **2''**, and also at least one outlet **4** for removing the reaction mixture,
   b) at least one heating apparatus for heating the inlet stream, line **2**, of the first prereactor **1**, or both,
   c) a second prereactor **19** comprising a hydrolysis catalyst and having at least one inlet for a fluid stream comprising at least partly the aqueous carboxylic acid from the bottom fraction or a fraction of the lower rectification zone **14** of the distillation column **13**, line **18**, also mixed with a carboxylic ester stream, line **20**, and at least one outlet, line **25**,
   d) a reactive distillation column **7** comprising a catalyst zone **8** having a hydrolysis catalyst and having an inlet **6** connected to the first prereactor **1**, an inlet connected to the second prereactor, line **25**, the catalyst zone **8** being disposed between a lower rectification zone **9** and an upper rectification zone **10**,
   e) line **22** attached at the distillation head of the reactive distillation column for removing the top fraction or as a purge,
   f) line **21** connected to the line **22** conducting the top fraction of the reactive distillation column **7** or to the upper rectification zone **10** or the condensation system of the reactive distillation column **7**, for removing distillate,
   g) line **11** attached at the distillation bottom of the reactive distillation column **7**, for removing the bottom fraction,
   h) a distillation column **13**, the line **11** forming the inlet for removing the bottom fraction of the reactive distillation column **7**, at whose distillation bottom, line **26**, aqueous carboxylic acid is removed,
   i) line **18** conducting a fraction of aqueous carboxylic acid withdrawn from the lower rectification zone **14** of the distillation column **13** or a portion of line **26**, this forming the inlet to the second prereactor **19**, mixed with the carboxylic ester, line **20**.

12. Apparatus according to Claim 11, **characterized in that** the line **18** for removing the aqueous carboxylic acid from the distillation column **13** is connected to a line **20** conducting a further carboxylic ester stream upstream of entry into the second prereactor **19**.

13. Apparatus according to at least one of Claims 11 and 12, **characterized in that** the line **21** conducting the carboxylic ester which is yet to be converted from the reactive distillation column **7** is connected to the inlet line **2** or one of the two process feeds, lines **2'** and **2''**, of the first prereactor **1**, which supply it with carboxylic ester and water.

14. Apparatus according to Claim 13, **characterized in that** the line **21** withdraws unconverted carboxylic ester from the lower half of the upper rectification column **10** of the reactive distillation column **7**.

15. Apparatus according to at least one of Claims 11 to 14, **characterized in that** the line **18** conducting the aqueous carboxylic ester stream is connected upstream of entry into the second prereactor **19** to the line **21** which conducts at least part of the top fraction or a fraction from the upper rectification zone **10** or the accompanying condensation system of the reactive distillation column **7**.

16. Apparatus according to at least one of Claims 11 to 15, **characterized in that** the reactive distillation column **7** has an inlet **6** for the feed stream from the first prereactor **1** in the upper third of the catalyst zone **8** or above the catalyst zone **8** and below the upper rectification zone **10**.

17. Apparatus according to at least one of Claims 11 to 16, **characterized in that** the reactive distillation column **7** has

**EP 1 565 241 B1**

an inlet **6** for the feed stream from the first prereactor **1** below the catalyst zone **8** and above the lower rectification zone **9** or in the upper third of the lower rectification zone **10**.

18. Apparatus according to at least one of Claims 11 to 17, **characterized in that** the reactive distillation column **7** has an inlet for the line **25** from the second prereactor **19** below the catalyst zone **8** and above the lower rectification zone **9** or in the upper third of the lower rectification zone **9**.

19. Apparatus according to at least one of Claims 11 to 18, **characterized in that** the hydrolysis catalysts in the first prereactor **1** or second prereactor **19** are each independently beds of a fixed catalyst, fixed in structured catalyst packings or installed in stirrers or are fluidized.

20. Apparatus according to at least one of Claims 11 to 19, **characterized in that** the hydrolysis catalyst in the reactive distillation column **7** is a bed of a solid catalyst, fixed in structured catalyst packing or arranged on the trays and also in the downcomers of a tray column.

**Revendications**

1. Procédé pour l'hydrolyse, catalysée par un acide, d'un ester d'acide carboxylique en l'acide carboxylique correspondant et l'alcool correspondant, en présence d'eau, dans lequel un courant d'entrée contenant l'ester d'acide carboxylique mélangé avec de l'eau est envoyé dans un premier pré-réacteur comportant un catalyseur d'hydrolyse, de sorte que l'ester d'acide carboxylique est au moins partiellement dissocié en les produits d'hydrolyse, le mélange réactionnel provenant de ce premier pré-réacteur est déchargé et au moins en partie envoyé dans une colonne de distillation réactive contenant un catalyseur d'hydrolyse, de sorte que le courant d'ester d'acide carboxylique contenant de l'eau est converti davantage en acide carboxylique et alcool et simultanément au moins partiellement fractionné en les composants, les composés plus volatils et les mélanges contenant l'ester d'acide carboxylique à bas point d'ébullition provenant au moins en partie de la zone de rectification supérieure de la colonne de distillation réactive et du système de condensation correspondant de la colonne de distillation réactive étant évacués sous forme de distillat et les composés moins volatils s'accumulant au moins en partie sous forme de fraction de bas de colonne qui est envoyée à une autre colonne de distillation, **caractérisé en ce que** l'acide carboxylique aqueux provenant de la fraction de bas de colonne ou provenant de la zone de rectification inférieure de la colonne de distillation, mélangé avec une nouvelle quantité d'ester d'acide carboxylique, est envoyé à un second pré-réacteur contenant un catalyseur d'hydrolyse, le mélange réactionnel provenant de ce second pré-réacteur est déchargé et au moins partiellement envoyé dans la zone de distillation réactive.

2. Procédé selon la revendication 1, **caractérisé en ce que** l'acide carboxylique aqueux provenant de la fraction de bas de colonne ou de la zone de rectification inférieure de la colonne de distillation est mélangé avec un courant d'ester d'acide carboxylique ou un courant contenant de l'ester d'acide carboxylique amené séparément, et ensuite envoyé au second pré-réacteur.

3. Procédé selon au moins l'une des revendications 1 et 2, **caractérisé en ce que** le distillat de la colonne de distillation réactive, contenant encore de l'ester d'acide carboxylique n'ayant pas réagi, est au moins en partie renvoyé au premier pré-réacteur.

4. Procédé selon au moins l'une des revendications 1 à 3, **caractérisé en ce que** l'acide carboxylique aqueux provenant de la fraction de bas de colonne ou de la zone de rectification inférieure de la colonne de distillation réactive est mélangé avec le distillat de la colonne de distillation réactive, contenant encore de l'ester d'acide carboxylique n'ayant pas réagi, et ensuite envoyé au second pré-réacteur.

5. Procédé selon au moins l'une des revendications 1 à 4, **caractérisé en ce que** le courant aqueux chaud d'acide carboxylique provenant de la fraction de bas de colonne ou de la zone de rectification inférieure de la colonne de distillation est utilisé pour le chauffage du courant contenant de l'ester d'acide carboxylique, à mélanger avec celui-ci.

6. Procédé selon au moins l'une des revendications 1 à 5, **caractérisé en ce que** le produit de réaction provenant du second pré-réacteur est introduit au-dessous de la zone de réaction et au-dessus de la zone de rectification inférieure ou dans le tiers supérieur de la zone de rectification inférieure de la colonne de distillation réactive.

7. Procédé selon au moins l'une des revendications 1 à 6, **caractérisé en ce que** l'ester d'acide carboxylique est choisi dans le groupe comprenant le formiate de méthyle et d'éthyle, l'acétate de méthyle, éthyle, propyle, isobutyle et tert-butyle, le propionate de méthyle et d'éthyle, ainsi que le butyrate de méthyle, éthyle et propyle.

8. Procédé selon au moins l'une des revendications 1 à 7, **caractérisé en ce que** le courant d'entrée sur le premier pré-réacteur contient l'azéotrope de l'ester d'acide carboxylique avec l'alcool correspondant.

9. Procédé selon au moins l'une des revendications 1 à 8, **caractérisé en ce que** les composés à bas point d'ébullition sont au moins en partie prélevés du distillat de la colonne de distillation réactive.

10. Procédé selon au moins l'une des revendications 1 à 9, **caractérisé en ce que** le procédé est conduit en continu.

11. Dispositif pour l'hydrolyse, catalysée par un acide, d'un ester d'acide carboxylique en l'acide carboxylique correspondant et l'alcool correspondant, en présence d'eau, conformément à un procédé selon au moins l'une des revendications 1 à 10, comprenant

    a) un premier pré-réacteur (1) contenant un catalyseur d'hydrolyse, avec au moins un conduit d'arrivée (2) pour l'introduction d'un courant de fluide contenant l'ester d'acide carboxylique, conduit (2'), et de l'eau, conduit (2"), ainsi qu'au moins une sortie (4) pour la décharge du mélange réactionnel,
    b) au moins un dispositif de chauffage pour le chauffage du courant d'entrée, conduit (2), du premier pré-réacteur (1) ou des deux,
    c) un second pré-réacteur (19) contenant un catalyseur d'hydrolyse, comportant au moins une entrée pour un courant de fluide contenant au moins en partie l'acide carboxylique aqueux provenant de la fraction de bas de colonne ou d'une fraction provenant de la zone inférieure ou d'une fraction provenant de la zone de rectification inférieure (14) de la colonne de distillation (13), conduit (18), en outre mélangé avec un courant d'ester d'acide carboxylique, conduit (20), et au moins une sortie, conduit (25),
    d) une colonne de distillation réactive (7) comprenant une zone de catalyseur (8) comportant un catalyseur d'hydrolyse avec une entrée (6) reliée au premier pré-réacteur (1), une entrée reliée au second pré-réacteur, conduit (25), la zone de catalyseur (8) étant placée entre une zone de rectification inférieure (9) et une zone de rectification supérieure (10),
    e) un conduit (22) raccordé à la tête de distillation de la colonne de distillation réactive, pour l'évacuation de la fraction de tête ou en tant que purge,
    f) un conduit (21) relié au conduit (22) conduisant la fraction de tête de la colonne de distillation réactive (7) ou à la zone de rectification supérieure (10) ou au système de condensation de la colonne de distillation réactive (7), pour l'évacuation du distillat,
    g) un conduit (11) raccordé au pied de distillation de la colonne de distillation réactive (7) pour le soutirage de la fraction dé bas de colonne,
    h) une colonne de distillation (13), le conduit (11) pour le soutirage de la fraction de bas de colonne de la colonne de distillation réactive (7) constituant l'entrée, au niveau du pied de distillation de laquelle, conduit (26) l'acide carboxylique aqueux est soutiré,
    i) un conduit (18) conduisant une fraction d'acide carboxylique aqueux, prélevé de la zone de rectification inférieure (14) de la colonne de distillation (13) ou quantité partielle du conduit (26), celle-ci, mélangée avec de l'ester d'acide carboxylique, conduit (20), constituant le flux arrivant au second pré-réacteur (19).

12. Dispositif selon la revendication 11, **caractérisé en ce que** le conduit (18) est relié à un conduit (20) conduisant un autre courant d'ester d'acide carboxylique, pour le soutirage de l'acide carboxylique aqueux hors de la colonne de distillation (13) avant l'entrée dans le second pré-réacteur (19).

13. Dispositif selon au moins l'une des revendications 11 et 12, **caractérisé en ce que** le conduit (21) de la colonne de distillation réactive (7), conduisant l'ester d'acide carboxylique n'ayant pas réagi, est relié au conduit d'entrée (2) ou à l'un des deux conduits d'alimentation de processus, conduits (2') et (2"), du premier pré-réacteur (1), qui alimente celui-ci en ester d'acide carboxylique et eau.

14. Dispositif selon la revendication 13, **caractérisé en ce que** le conduit (21) prélève l'ester d'acide carboxylique n'ayant pas réagi de la moitié inférieure de la zone de rectification supérieure (10) de la colonne de distillation réactive (7).

15. Dispositif selon au moins l'une des revendications 11 à 14, **caractérisé en ce que** le conduit (18) conduisant le courant aqueux d'acide carboxylique est relié, avant l'entrée dans le second pré-réacteur (19), au conduit (21) qui conduit au moins en partie la fraction de tête ou une fraction provenant de la zone de rectification supérieure (10) ou du système de condensation correspondant de la colonne de distillation réactive (7).

**16.** Dispositif selon au moins l'une des revendications 11 à 15, **caractérisé en ce que** la colonne de distillation réactive (7) comporte une entrée (6) pour l'affluent provenant du premier pré-réacteur (1) dans le tiers supérieur de la zone de catalyseur (8) ou au-dessus de la zone de catalyseur (8) et au-dessous de la zone de rectification supérieure (10).

**17.** Dispositif selon au moins l'une des revendications 11 à 16, **caractérisé en ce que en ce que** la colonne de distillation réactive (7) comporte une entrée (6) pour le courant d'arrivée provenant du premier pré-réacteur (1) au-dessous de la zone de catalyseur (8) et au-dessus de la zone de rectification inférieure (9) ou dans le tiers supérieur de la zone de rectification inférieure (10).

**18.** Dispositif selon au moins l'une des revendications 11 à 17, **caractérisé en ce que en ce que** la colonne de distillation réactive (7) comporte une entrée pour le conduit (25) du second pré-réacteur (19) au-dessous de la zone de catalyseur (8) et au-dessus de la zone de rectification inférieure (9) ou dans le tiers supérieur de la zone de rectification inférieure (9).

**19.** Dispositif selon au moins l'une des revendications 11 à 18, **caractérisé en ce que** dans le premier pré-réacteur (1) ou le second pré-réacteur (19) les catalyseurs d'hydrolyse sont des lits, indépendants les uns des autres, d'un catalyseur solide, fixés dans des garnissages texturés de catalyseur ou fluidisés ou introduits dans des mécanismes d'agitation.

**20.** Dispositif selon au moins l'une des revendications 11 à 19, **caractérisé en ce que** le catalyseur d'hydrolyse dans la colonne de distillation réactive (7) est un lit d'un catalyseur solide, fixé en garnissage texturé de catalyseur ou disposé sur les plateaux ainsi que dans les corps d'écoulement d'une colonne à plateaux.

Fig. 1

EP 1 565 241 B1

Fig. 2

EP 1 565 241 B1

Fig. 3

Fig. 4

EP 1 565 241 B1

Fig. 5